# EUROPEAN PATENT APPLICATION

(11) **EP 4 458 844 A1**
(43) Date of publication of application: **06.11.2024**
(21) Application number: 22913024.0
(22) Date of filing: 31.03.2022
(51) Int. Cl.: C07K 14/165, C12N 15/50, A61K 39/215, A61P 31/14

(54) **ANTI-CORONAVIRUS POLYPEPTIDE, AND DERIVATIVES THEREOF AND APPLICATION THEREOF**

(30) Priority: 31.12.2021 CN 202111675955
(71) Applicant: Institute Of Microbiology, Chinese Academy Of Sciences, Beijing 100101 (CN); Hybio Pharmaceutical Co., Ltd., Shenzhen, Guangdong 518057 (CN)
(72) Inventor: GAO, Fu, Beijing 100101 (CN); ZENG, Shaogui, Beijing 100101 (CN); WANG, Qihui, Beijing 100101 (CN); TANG, Yangming, Beijing 100101 (CN); CHEN, Jiantao, Beijing 100101 (CN); WU, Lili, Beijing 100101 (CN); ZHENG, Anqi, Beijing 100101 (CN)
(74) Representative: Fabry, Bernd
(86) International application number: PCT/CN2022/084473
(87) International publication number: WO 2023/123722

(57) **Abstract**

The invention relates to an anti-coronavirus polypeptide as well as derivatives and application thereof, provides the anti-coronavirus polypeptide, and provides cholesterol-containing derivatives of the polypeptide on the basis of the anti-coronavirus polypeptide. Particularly, the original strain and the variant strain of the SARS-CoV-2 have an unexpected inhibition effect, can be used for preparing medicines or vaccines for preventing or treating the new coronavirus, and have great prevention or treatment potential.

## Description

### TECHNICAL FIELD

The present invention relates to the field of biopharmaceuticals, and in particular, to a polypeptide, a derivative thereof, a polynucleotide encoding the polypeptide, a nucleic acid construct comprising the polynucleotide, an expression vector comprising the nucleic acid construct, a transformed cell, and a pharmaceutical composition comprising same for preventing or treating a disease caused by a coronavirus, and use thereof in preparing a medicament or a vaccine for preventing and/or treating COVID-19.

### BACKGROUND

Coronaviruses are enveloped viruses with a linear positive-sense single-stranded antisense RNA genome, and consist of a large group of viruses widely present in nature. Some coronaviruses may infect humans and cause diseases, and seven coronaviruses are found capable of infecting humans so far, including SARS-CoV, MERS-CoV, SARS-CoV-2, HCoV-229E, HCoV-OC43, HCoV-NL63, and HCoV-HKU1.

SARS-CoV-2 is the pathogen of the COVID-19 pandemic, and is the third coronavirus causing human epidemics since the 21^{st} century. Currently, although a variety of vaccines and monoclonal antibody therapies against SARS-CoV-2 have been approved for emergency, many studies have shown that they exhibit reduced protective effects against currently widely spread new coronavirus variants, such as Beta, Delta, Omicron, and the like. Thus there's an urgent need for the development of broad-spectrum therapies or vaccines against the new variants that are currently prevalent and may emerge in the future. At present, a number of coronaviruses related to SARS-CoV-2 (Sarbecovirus species) have been reported, such as bat-derived RaTG13, RmYN02, ZC45, ZXC21, etc., and pangolin-derived GX/P2V/2017 and GD/1/2019, and studies have shown that RaTG13, GX/P2V/2017, GD/1/2019, and the like also possess the potential of infecting humans. Accordingly, the development of therapies or vaccines against such related coronaviruses is urgently needed so as to cope with underlying epidemics in the future.

### SUMMARY

In view of the defects of the prior art, the present invention is intended to provide a polypeptide, a polynucleotide encoding same, a nucleic acid construct comprising the polynucleotide, an expression vector comprising the nucleic acid construct, a transformed cell, and a pharmaceutical composition comprising same for preventing or treating a disease caused by a coronavirus, and use thereof in preparing a medicament or a vaccine for preventing or treating a disease caused by a coronavirus.

In a first aspect, the present invention provides an anti-coronavirus polypeptide, wherein the anti-coronavirus polypeptide is a polypeptide having any one of the following sequences (1) to (8):
(1) a polypeptide, comprising the amino acid sequence set forth in SEQ ID NO: 1;
(2) a polypeptide, comprising the amino acid sequence set forth in SEQ ID NO: 2;
(3) a polypeptide, comprising the amino acid sequence set forth in SEQ ID NO: 3;
(4) a polypeptide, comprising the amino acid sequence set forth in SEQ ID NO: 12;
(5) a polypeptide, comprising the amino acid sequence set forth in SEQ ID NO: 13;
(6) a polypeptide, comprising the amino acid sequence set forth in SEQ ID NO: 14;
(7) a polypeptide, comprising an amino acid sequence having 1 or more amino acid residue substitutions, deletions, additions, or insertions compared with any one of the amino acid sequences of the polypeptides (1) to (6), and having an inhibitory activity against a coronavirus; or
(8) a polypeptide, comprising an amino acid sequence having at least 60%, 65%, 70%, 75%, 80%, 85%, 90%, 93%, 94%, or 95% identity to any one of the amino acid sequences of the polypeptides (1) to (6), and having an inhibitory activity against a coronavirus.

Preferably, the sequence of the polypeptide is SEQ ID NO: 1, SEQ ID NO: 2, SEQ ID NO: 3, SEQ ID NO: 12, SEQ ID NO: 13, or SEQ ID NO: 14.

In a second aspect, the present invention provides an anti-coronavirus polypeptide derivative, wherein the polypeptide derivative is the anti-coronavirus polypeptide described above modified by a cholesterol derivative.

Furthermore, the site of the cholesterol derivative modification is at the C-terminus or N-terminus or on a basic amino acid side chain of the polypeptide. Preferably, the site of the cholesterol derivative modification is at the C-terminus of the polypeptide.

Furthermore, the cholesterol derivative is linked to the C-terminus of the polypeptide via a linker or directly.

Furthermore, the cholesterol derivative is a derivative of cholesterol modified by cysteine and/or PEG.

Furthermore, the cholesterol derivative is selected from 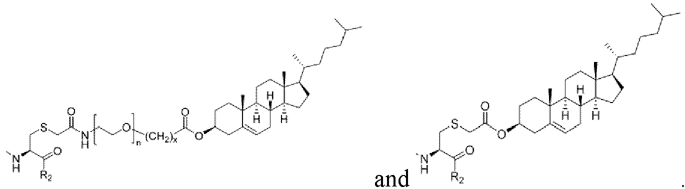
R₂ is OH or NH₂.
n is any integer of 1-20, preferably any integer of 2-6, for example, 1, 2, 3, 4, 5, 6, 7, 8, 9, 10, 11, 12, 13, 14, 15, 16, 17, 18, 19, or 20.
x is any integer of 1-6, for example, 1, 2, 3, 4, 5, or 6.
Furthermore, the linker is -NH-PEGn-(CH₂)x-CO-, (GSG)m, (GSGSG)m, (GSG)m-CONH-PEGn-(CH₂)x-CO-, or (GSGSG)m-CONH-PEGn-(CH₂)x-CO-.
m is any integer of 0-6, for example, 0, 1, 2, 3, 4, 5, or 6.
n is any integer of 1-20, preferably any integer of 2-6, for example, 1, 2, 3, 4, 5, 6, 7, 8, 9, 10, 11, 12, 13, 14, 15, 16, 17, 18, 19, or 20.
x is any integer of 1-6, for example, 1, 2, 3, 4, 5, or 6.

Furthermore, the polypeptide derivative has a structural formula represented by the following structure I, structure II, and structure III: 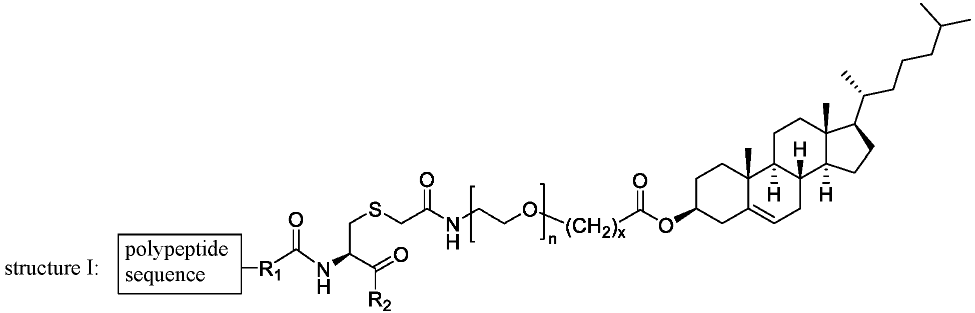 structure I: polypeptide sequence -R1-Cys(CH₂CONH(PEG)ₙ(CH₂)ₓCOOChol)-R₂ 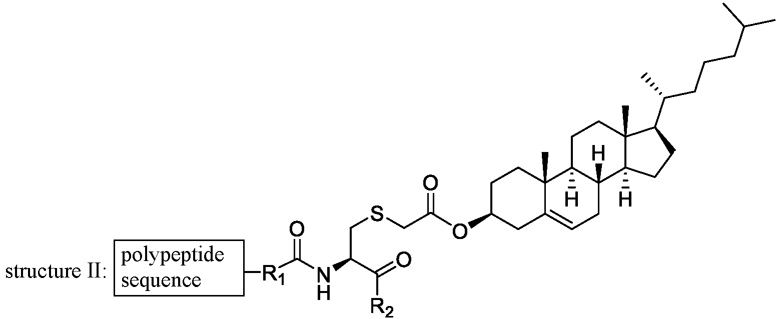 structure II: polypeptide sequence -R₁-Cys(CH₂COOChol)-R₂ 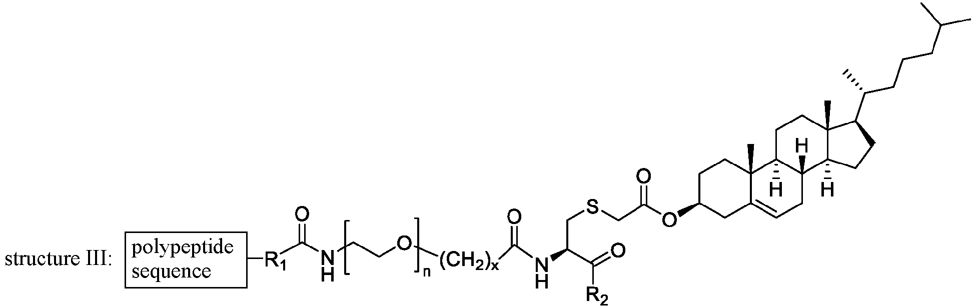 structure III: polypeptide sequence -R1-CONH(PEG)ₙ(CH₂)ₓ-Cys(CH₂COOChol)-R₂

The polypeptide sequence refers to the polypeptide sequence set forth in any one of (1) to (8) above;
Preferably, the polypeptide sequence is SEQ ID NO: 1, SEQ ID NO: 2, SEQ ID NO: 3, SEQ ID NO: 12, SEQ ID NO: 13, or SEQ ID NO: 14.

R₁ is (GSG)m or (GSGSG)m; m is any integer of 0-6, and preferably m is 1 or 2;
R₂ is OH or NH₂; preferably, R₂ is NH₂.

Furthermore, the polypeptide derivative is selected from:

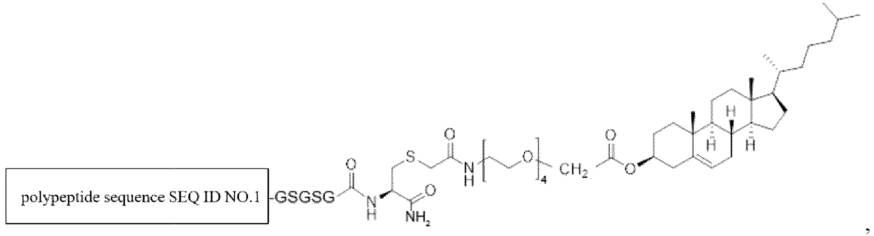

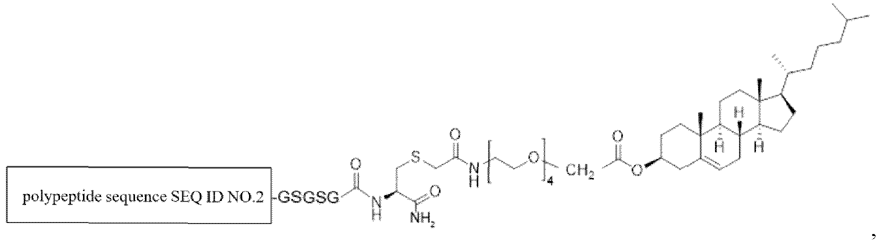

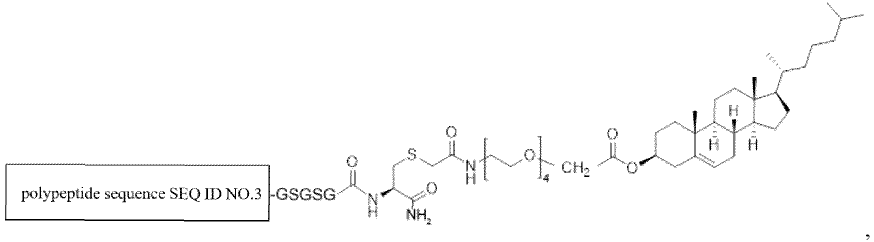

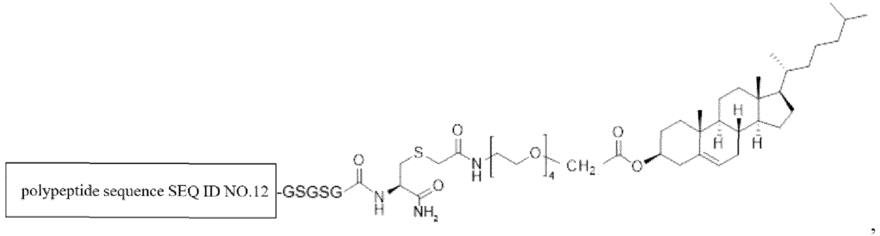

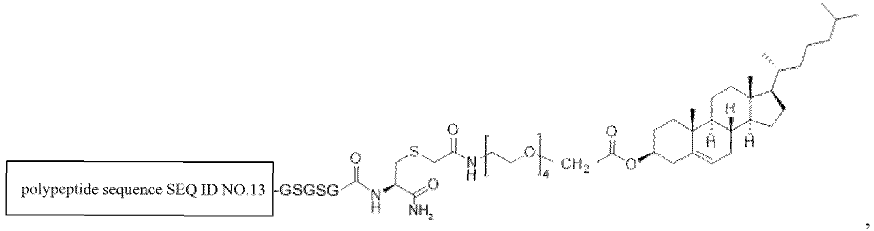

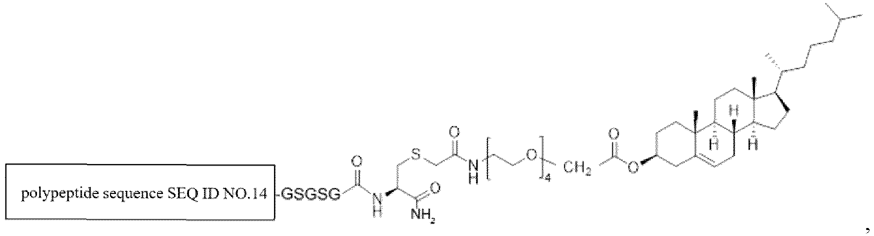

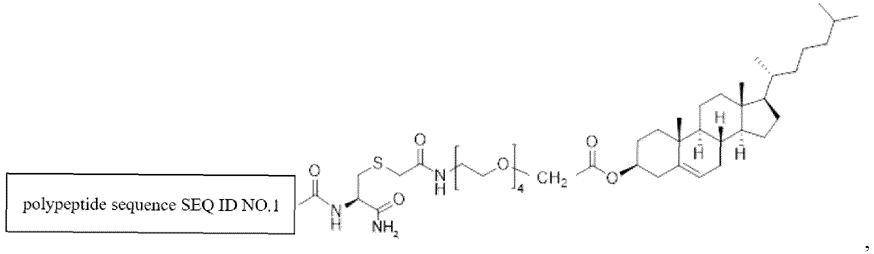

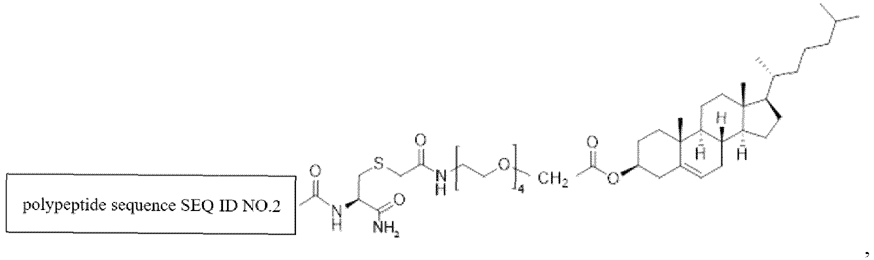

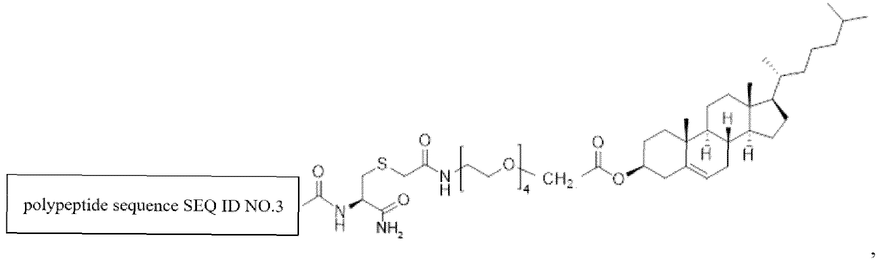

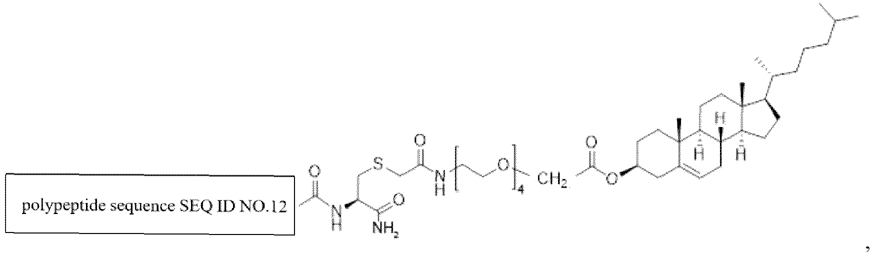

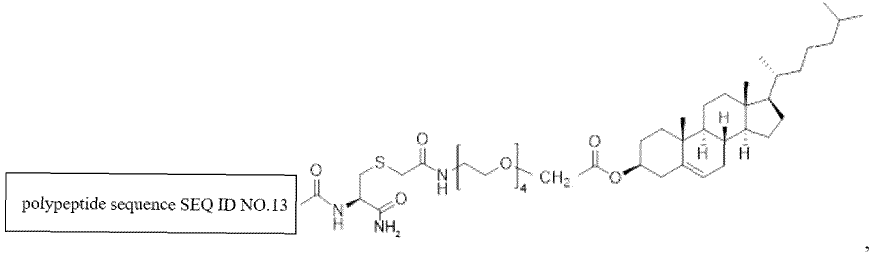

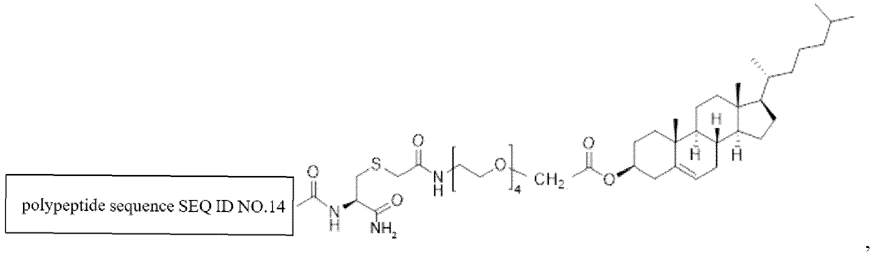

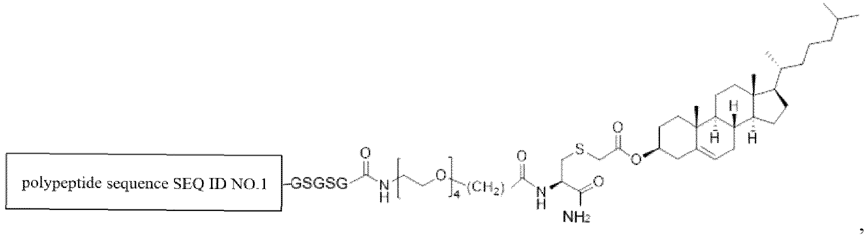

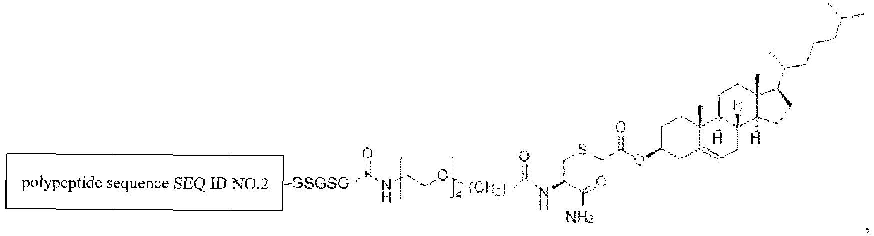

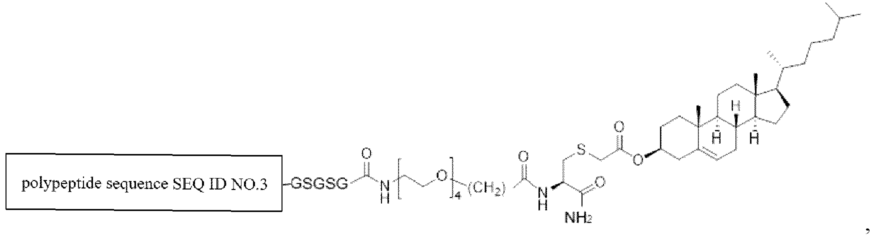

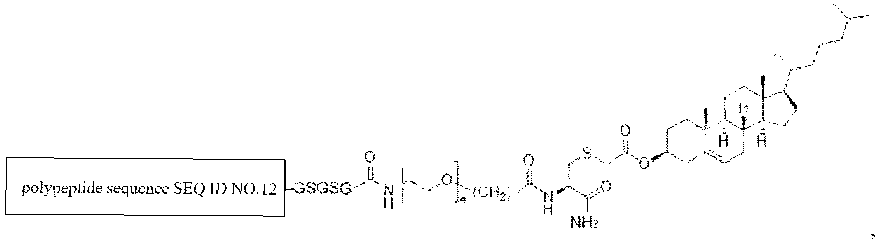

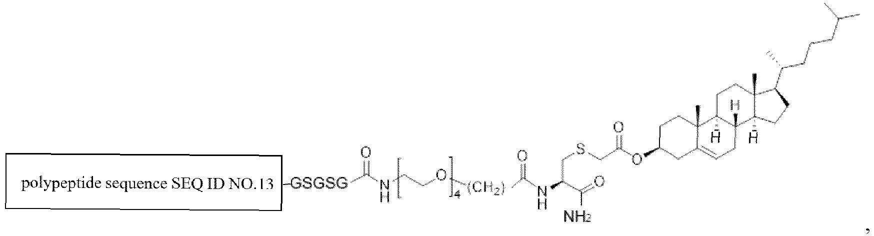

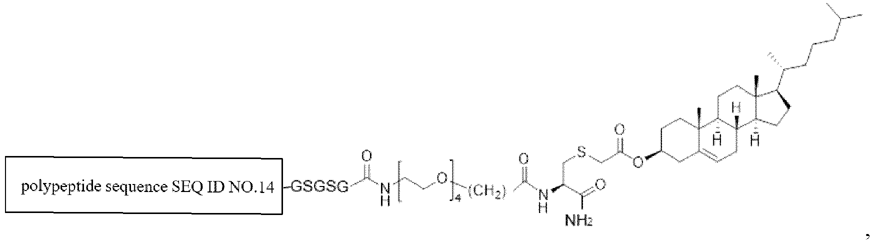

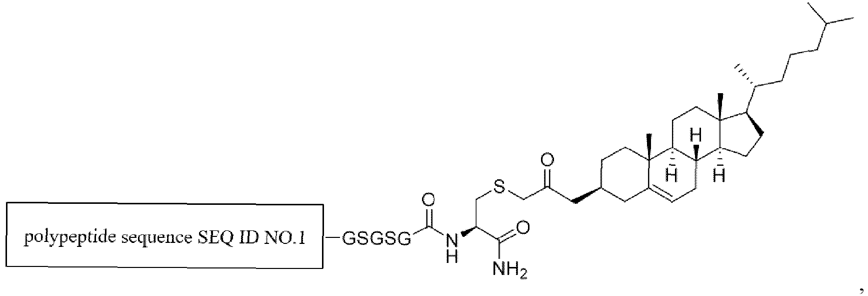

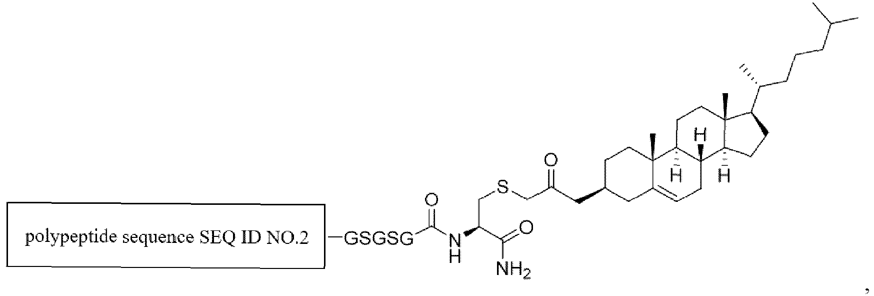

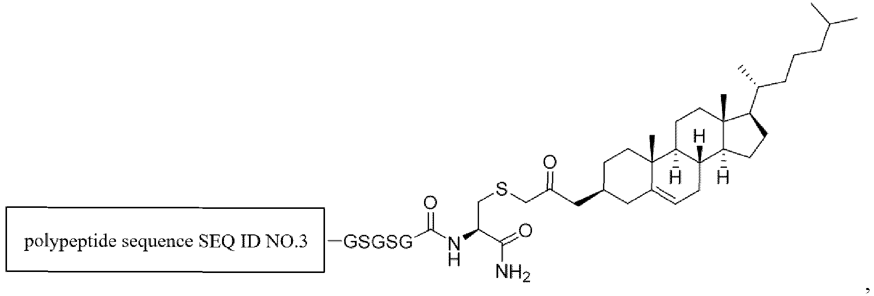

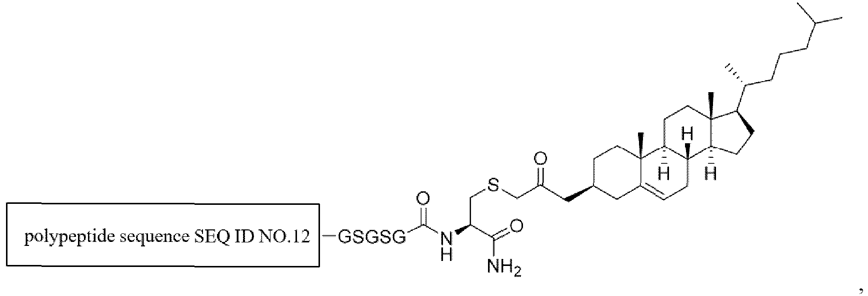

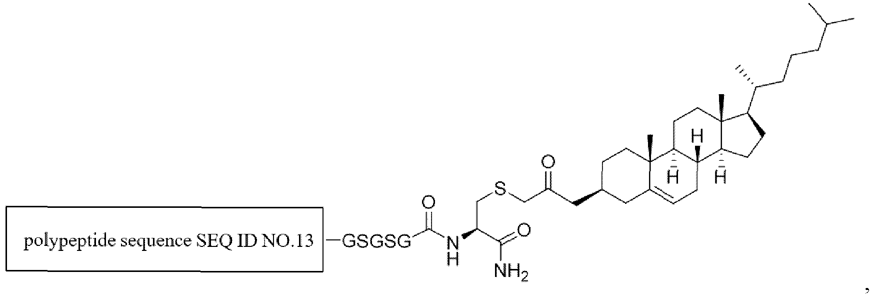

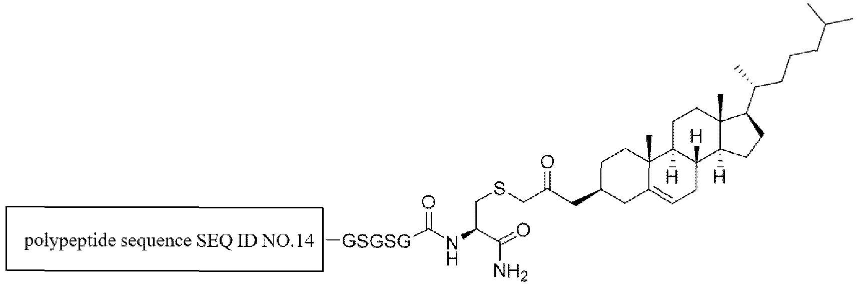

In a third aspect, the present invention provides a polynucleotide sequence encoding the polypeptide according to the first aspect.

In a fourth aspect, the present invention provides a vector, comprising the polynucleotide sequence according to the third aspect.

In a fifth aspect, the present invention provides a cell, comprising the vector according to the fourth aspect of the present invention.

In a sixth aspect, the present invention provides a pharmaceutical composition, comprising the polypeptide according to the first aspect and/or the derivative according to the second aspect, and a pharmaceutically acceptable carrier and/or excipient.

In one embodiment, the pharmaceutical composition is in the form of an inhalant, a nasal formulation, an oral formulation, or a parenteral formulation.

Preferably, the oral formulation is selected from a tablet, a capsule, a granule, a suspension, a pill, and a solution.

Preferably, the parenteral formulation is an injectable or bolus injection formulation.

More preferably, the pharmaceutical composition is a vaccine composition.

Preferably, the dosage form of the medicament is tablet, capsule, dripping pill, aerosol, pill, powder, solution, suspension, emulsion, granule, liposome, transdermal agent, suppository, or lyophilized powder for injection.

Preferably, the medicament is preferably administered by: injection, including subcutaneous injection, intravenous injection, intramuscular injection, intraperitoneal injection, intracisternal injection or infusion, etc.; intraluminal administration, e.g., intrarectal, intravaginal and sublingual administration; respiratory administration, e.g., nasal administration; mucosal administration; or topical administration.

In a seventh aspect, the present invention provides use of the polypeptide, the polypeptide derivative, or the fusion protein or conjugate above in preparing a medicament for inhibiting a coronavirus or in treating and/or preventing a disease caused by a coronavirus.

Preferably, the coronavirus is selected from the SARS-CoV, MERS-CoV, SARS-CoV-2, HCoV-229E, HCoV-OC43, HCoV-NL63, or HCoV-HKU1 prototype or a variant thereof.

Preferably, the coronavirus is selected from the SARS-CoV-2 prototype and a SARS-CoV-2 variant.

More preferably, the SARS-CoV-2 variant is selected from Alpha, Beta, Gamma, Delta, Omicron, Kappa, Lambda, and Deltacron.

Preferably, the disease caused by a coronavirus is selected from corona virus disease 2019 (COVID-19), severe acute respiratory syndrome, and Middle East respiratory syndrome.

In an eighth aspect, the present invention provides a method for inhibiting a coronavirus *in vitro*, comprising: administering the polypeptide, the polypeptide derivative, the fusion protein or conjugate, or the pharmaceutical composition above.

Preferably, the coronavirus is the SARS-CoV-2 prototype and/or a SARS-CoV-2 variant.

More preferably, the variant includes Alpha, Beta, Gamma, Delta, and Omicron.

Furthermore, the administration comprises contacting the polypeptide according to the first aspect, the derivative according to the second aspect, and/or the pharmaceutical composition according to the third aspect with the coronavirus.

In a ninth aspect, the present invention provides a method for preventing or treating a coronavirus infection or a disease caused by a coronavirus in a subject, comprising: administering to the subject the polypeptide, the polypeptide derivative, the fusion protein or conjugate, or the pharmaceutical composition above.

Preferably, the coronavirus is the SARS-CoV-2 prototype and/or a SARS-CoV-2 variant. More preferably, the SARS-CoV-2 variant includes Alpha, Beta, Gamma, Delta, Omicron, Kappa, Lambda, and Deltacron.

In a tenth aspect, the present invention provides the polypeptide, the polypeptide derivative, and the fusion protein or conjugate above for use in preventing or treating a coronavirus infection or a disease caused by a coronavirus in a subject.

Preferably, the coronavirus is the SARS-CoV-2 prototype and/or a SARS-CoV-2 variant.

More preferably, the variant includes Alpha, Beta, Gamma, Delta, Omicron, Kappa, Lambda, and Deltacron.

The present invention has the following beneficial effects:
The present invention designs a polypeptide P3 based on the HR2 region of the S protein of SARS-CoV-2. The present invention acquires the polypeptides of interest by a series of engineering procedures (including one or more amino acid additions or substitutions), and modifies the polypeptides with a linker and cholesterol to give polypeptide derivatives. The polypeptides and the derivatives thereof exhibit good inhibitory effects against the SARS-CoV-2 prototype and variants Alpha, Beta, Gamma, Delta and Omicron, and thus possess great therapeutic and prophylactic prospects in preparing therapies and vaccines for preventing and treating COVID-19.

### BRIEF DESCRIPTION OF THE DRAWINGS

FIG. 1 shows the inhibitory effects of different polypeptides on the pseudovirus of the SARS-CoV-2 prototype according to Example 2;
FIG. 2 shows the inhibitory effects of different polypeptides on the pseudovirus of SARS-CoV-2 variant Omicron according to Example 2;
FIG. 3 shows the inhibitory effects of polypeptide P3-2 on the pseudoviruses of the SARS-CoV-2 prototype and variants Alpha, Beta, Gamma, Delta and Omicron according to Example 3;
FIG. 4 shows the inhibitory effects of polypeptide P3-2-GS-Chol on the pseudoviruses of the SARS-CoV-2 prototype and variants Alpha, Beta, Gamma, Delta and Omicron according to Example 3;
FIG. 5 shows the inhibitory effects of polypeptide P3-2-PEG-Chol on the pseudoviruses of the SARS-CoV-2 prototype and variants Alpha, Beta, Gamma, Delta and Omicron according to Example 3;
FIG. 6 shows the inhibitory effects of polypeptide P3-2-GS-PEG-Chol on the pseudoviruses of the SARS-CoV-2 prototype and variants Alpha, Beta, Gamma, Delta and Omicron according to Example 3;
FIG. 7 shows the inhibitory effects of polypeptide P3-1-GS-PEG-Chol on the pseudoviruses of the SARS-CoV-2 prototype and variants Alpha, Beta, Gamma, Delta and Omicron according to Example 3;
FIG. 8 shows the inhibitory effects of truncated polypeptides obtained by continuous single amino acid deletions at the N-terminus of polypeptides P3-2-GS-Chol and P3-2-GS-PEG-Chol on the pseudovirus of SARS-CoV-2 prototype according to Example 4;
FIG. 9 shows the inhibitory effects of truncated polypeptides obtained by continuous single amino acid deletions at the N-terminus of polypeptides P3-2-GS-Chol and P3-2-GS-PEG-Chol on the pseudovirus of variant Delta according to Example 4;
FIG. 10 shows the inhibitory effects of truncated polypeptides obtained by continuous single amino acid deletions at the N-terminus of polypeptides P3-2-GS-Chol and P3-2-GS-PEG-Chol on the pseudovirus of variant Omicron according to Example 4;
FIG. 11 shows the inhibitory effects of polypeptide P3-2-GS-chol on live SARS-CoV-2 prototype and variants thereof in Vero cells according to Example 5;
FIG. 12 shows the inhibitory effects of polypeptide P3-2-PEG-chol on live SARS-CoV-2 prototype and variants thereof in Vero cells according to Example 5;
FIG. 13 shows the inhibitory effects of polypeptide P3-2-GS-PEG-chol on live SARS-CoV-2 prototype and variants thereof in Vero cells according to Example 5;
FIG. 14 shows the inhibitory effects of polypeptide P3-2-GS-chol on live SARS-CoV-2 prototype and variants thereof in Vero E6 cells according to Example 6;
FIG. 15 shows the inhibitory effects of polypeptide P3-2-PEG-chol on live SARS-CoV-2 prototype and variants thereof in Vero E6 cells according to Example 6;
FIG. 16 shows the inhibitory effects of polypeptide P3-2-GS-PEG-chol on live SARS-CoV-2 prototype and variants thereof in Vero E6 cells according to Example 6.

### DETAILED DESCRIPTION

The coronavirus spike (S) protein plays an important role in mediating virus invasion and consists of two subunits S1 and S2. S1 is responsible for recognizing the receptor, and S2 mediates membrane fusion of the viral envelope with the host cell membrane. Heptapeptide repeats HR1 and HR2 in S2 exert their membrane fusion functionality by forming a six-helix bundle structure. Studies have shown that the addition of exogenous HR1 or HR2 polypeptide can inhibit the formation of HR1 and HR2 six-helix bundle structures in the virus, thereby suppressing the membrane fusion process.

The present invention is intended to provide a group of polypeptide entry inhibitors having inhibitory functionality against SARS-CoV-2 and SARS-like virus infections. The polypeptide entry inhibitors bind to the HR1 region in SARS-CoV-2 and SARS-like virus S2 protein to interfere with the formation process of the virus six-helix structure, thereby suppressing the fusion and infection procedures of the virus.

The present invention designs a polypeptide P3 based on the HR2 region of the S protein of SARS-CoV-2. The present invention acquires polypeptides P3-1, P3-2, and P3-3 by a series of engineering procedures (including one or more amino acid additions or substitutions), and modifies the polypeptides P3-1, P3-2, and P3-3 with a linker and cholesterol. The cholesterol-modified polypeptide derivatives exhibit good inhibitory effects against the SARS-CoV-2 prototype and variant Omicron, and can thus be used for preparing medicament and vaccines for preventing and treating COVID-19.

The specific sequences of the polypeptides are shown in Table 1 or detailed in polypeptide sequence listing. The polypeptides of the present invention exhibit a surprisingly high inhibitory activity against SARS-CoV-2, especially for variant Omicron.

In the present invention, the polypeptide may be a polypeptide comprising SEQ ID NOs: 1-6 or a variant thereof.

For example, the polypeptide may comprise an amino acid sequence having 1 or more amino acid substitutions, additions, deletions, or insertions compared with the amino acid sequence set forth in SEQ ID NO: 1, 2, or 3.

The amino acid addition refers to the addition of an amino acid at the C-terminus or N-terminus of an amino acid sequence, e.g., SEQ ID NO: 1, 2, or 3, as long as the polypeptide retains the inhibitory activity against the coronavirus.

The amino acid substitution refers to the substitution of a certain amino acid residue at a certain position in an amino acid sequence, e.g., the sequence of SEQ ID NO: 1, 2, or 3, with another amino acid residue, as long as the polypeptide retains inhibitory activity against the coronavirus.

The amino acid insertion refers to the insertion of amino acid residues at proper positions in an amino acid sequence, e.g., the sequence of SEQ ID NO: 1, 2, or 3, where all or part of the inserted amino acid residues may be adjacent, or none of the inserted amino acids may be adjacent, as long as the polypeptide retains inhibitory activity against the coronavirus.

The amino acid deletion refers to the deletion of 1, 2, 3 or more amino acids in an amino acid sequence, e.g., the sequence of SEQ ID NO: 1, 2, or 3, as long as the polypeptide retains inhibitory activity against the coronavirus.

The polypeptide or polypeptide derivative of the present invention can be produced synthetically, or the polypeptide of the present invention may be expressed in a cell. For example, the polypeptide of the present invention can be synthesized by chemical means.. Alternatively, the polypeptide of the present invention may be expressed in a recombinant cell. The type of the cell is not limited. For example, the cell may be a eukaryotic cell or a prokaryotic cell. The eukaryotic cell may be a fungal cell (e.g., a yeast cell), an insect cell, or a mammalian cell (e.g., a mouse cell). The prokaryotic cell may be a bacterial cell, e.g., an *Escherichia coli* cell.

The cholesterol modification of the polypeptide of the present invention may be conducted at the C-terminus of the polypeptide, such that the polypeptide is linked to the cholesterol moiety at the C-terminus. The linkage can be a direct linkage or can be a linkage via a linker. Methods for cholesterol modification in the polypeptide of the present invention are well known in the art.

The identity and length of the linker can vary. For example, the linker can be (GSG)m or (GSGSG)m, where m can be any integer, e.g., 1, 2, 3, 4, 5, 6, etc.

The linker can be PEGylated. Means for PEGylation modification are known to those skilled in the art. As used herein, the term PEGylated linker refers to a linker to which one or more PEGs are attached. As used herein, the PEGylated linker can be PEGylated (GSG)n or (GSGSG)n. n can be any integer, e.g., 1, 2, 3, 4, 5, 6, etc.

As used herein, the "PEGm" means that the number of polyethylene glycol repeat units is m. m can be any integer, e.g., 1, 2, 3, 4, 5, 6, 8, 10, 12, 14, 16, 18, or 20.

As used herein, the coronavirus can be any coronavirus. Preferably, the coronavirus is a prototype or a variant Alpha, Beta, Gamma, Delta, Omicron, Kappa, Lambda, or Deltacron. The polypeptide or polypeptide derivative can be formulated into a medicament or a pharmaceutical composition, and the pharmaceutical composition is in the form of an inhalant, a nasal formulation, an oral formulation, or a parenteral formulation.

Preferably, the oral formulation is selected from a tablet, a capsule, a granule, a suspension, a pill, and a solution.

Preferably, the parenteral formulation is an injectable or bolus injection formulation.

Preferably, the pharmaceutical composition is a vaccine composition.

The medicament can be a tablet, a capsule, a dripping pill, an aerosol, a pill, a powder, a solution, a suspension, an emulsion, a granule, a liposome, a transdermal agent, a suppository, or a lyophilized powder for injection. Such medicaments or pharmaceutical compositions may be administered by various modes of administration, such as injection, including subcutaneous injection, intravenous injection, intramuscular injection, intraperitoneal injection, intracisternal injection or infusion, etc.; intraluminal administration, e.g., intrarectal, intravaginal and sublingual administration; respiratory administration, e.g., nasal administration; mucosal administration; or topical administration.

The present invention provides a method for preventing or treating a coronavirus infection or a disease caused by a coronavirus in a subject, comprising: administering the polypeptide or the polypeptide derivative of the present invention. The present invention also provides a polypeptide or a polypeptide derivative for use in preventing or treating a coronavirus infection or a disease caused by a coronavirus in a subject.

The present invention also relates to a method for preventing or treating a coronavirus infection or a disease caused by a coronavirus in a subject, comprising: administering to the subject/patient a composition of the polypeptide or the polypeptide derivative of the present invention.

Preferably, the coronavirus is a prototype or a variant Alpha, Beta, Gamma, Delta, Omicron, Kappa, Lambda, or Deltacron.

The present invention also relates to a polypeptide or a polypeptide derivative, or a composition for use in preventing or treating a coronavirus infection or a disease caused by a coronavirus in a subject, wherein preferably, the coronavirus is a prototype or a variant Alpha, Beta, Gamma, Delta, Omicron, Kappa, Lambda, or Deltacron.

The present invention also relates to a polypeptide or a polypeptide derivative, or a composition, a pharmaceutical composition or a kit thereof for use in preventing or treating a coronavirus infection or a disease caused by a coronavirus in a subject/patient, wherein preferably, the coronavirus is a prototype or a variant Alpha, Beta, Gamma, Delta, Omicron, Kappa, Lambda, or Deltacron.

### Examples

In order to understand the present invention more clearly, the present invention will be further described with reference to the following examples and drawings. The examples are given for the purpose of illustration only and are not intended to limit the present invention in any way. In the examples, all of the reagents and starting materials are commercially available, and the experimental methods without specifying the specific conditions are conventional methods with conventional conditions well known in the art, or conditions suggested by the instrument manufacturer.

The present invention will be described in detail below.

The present invention designs a polypeptide P3 based on the HR2 region of the S protein of SARS-CoV-2, and modifies the polypeptide P3 by a series of engineering procedures (including one or more amino acid additions or substitutions) to give three modified polypeptides designated as P3-1, P3-2, and P3-3. The polypeptides P3-1, P3-2, and P3-3 are further modified with a linker and cholesterol to give corresponding cholesterol-modified polypeptide derivatives, which can be obtained by conventional synthetic methods in the art.

### Instruments and materials:

Pseudovirus packaging virus G*VSV-delG (purchased from BrainVTA (Wuhan) Co., Ltd).

Eukaryotic protein pCAGGS expression vector (supplied by Suzhou GENEWIZ).

HEK293T ACE2 cell (purchased from Suzhou GENEWIZ).

### Polypeptide:

Polypeptide P3 was designed by homology comparison of HR1 and HR2 sequences in human infectious coronavirus proteins and modified by a series of engineering procedures (including one or more amino acid additions or substitutions) to give three modified polypeptides designated as P3-1, P3-2, and P3-3. The polypeptides P3-1, P3-2, and P3-3 were further modified with a linker and cholesterol to give corresponding cholesterol-modified polypeptide derivatives. The specific sequences and structures of the polypeptides and the polypeptide derivatives are shown in Table 1. The polypeptides and the polypeptide derivatives were synthesized and purified by Hybio Pharmaceutical Co., Ltd., with a purity of >95%.

**Table 1. Amino acid sequences used in the examples**

| | |
|---|---|
| **P3** | ISGINASVVNIQKEIDRLNEVAKNLNESLIDLQEL SEQ ID NO.4 |
| **P3-1** | ISGINASVVNIQKEIDRLNEVAKNLNESLIDLKEL SEQ ID NO. 1 |
| **P3-2** | VDLGDISGINASVVNIQKEIDRLNEVAKNLNESLIDLQEL SEQ ID NO.2 |
| **P3-3** | VKFGDISGINASVVNIKEEIDRLYEVVKNLNESLIDLQEL SEQ ID NO.3 |
| **P3-1-GS-Chol** | ISGINASVVNIQKEIDRLNEVAKNLNESLIDLKEL**GSGSG**C(CH₂COOChol)-NH₂, wherein the polypeptide sequence is SEQ ID NO: 5 |
| **P3-2-GS-Chol** | VDLGDISGINASVVNIQKEIDRLNEVAKNLNESLIDLQEL**GSGSG**C(CH₂CO OChol)-NH₂, wherein the polypeptide sequence is SEQ ID NO: 6 |
| **P3-3-GS-Chol** | VKFGDISGINASVVNIKEEIDRLYEVVKNLNESLIDLQEL**GSGSG**C(CH₂COO Chol)-NH₂, wherein the polypeptide sequence is SEQ ID NO: 7 |
| **P3-4-GS-Chol** | SVVNIKEEIDRLYEVVKNLNESLIDLQEL**GSGSG**C(CH₂COOChol)-NH₂, wherein the polypeptide sequence is SEQ ID NO: 8 |
| **P3-1-PEG-Chol** | ISGINASVVNIQKEIDRLNEVAKNLNESLIDLKELC(CH₂CONH(PEG)₄CH₂CO OChol)-NH₂, wherein the polypeptide sequence is SEQ ID NO: 9 |
| **P3-2-PEG-Chol** | VDLGDISGINASVVNIQKEIDRLNEVAKNLNESLIDLQELC(CH₂CONH(PEG) ₄CH₂COOChol)-NH₂, wherein the polypeptide sequence is SEQ ID NO: 10 |
| **P3-3-PEG-Chol** | VKFGDISGINASVVNIKEEIDRLYEVVKNLNESLIDLQELC(CH₂CONH(PEG) ₄CH₂COOChol)-NH₂, wherein the polypeptide sequence is SEQ ID NO: 11 |
| **P3-1-GS-PEG-Chol** | ISGINASVVNIQKEIDRLNEVAKNLNESLIDLKEL**GSGSG**C(CH₂CONH(PEG) ₄CH₂COOChol)-NH₂, wherein the polypeptide sequence is SEQ ID NO: 5 |
| **P3-2-GS-PEG-Chol** | VDLGDISGINASVVNIQKEIDRLNEVAKNLNESLIDLQEL**GSGSG**C(CH₂CO NH(PEG)₄CH₂COOChol)-NH₂, wherein the polypeptide sequence is SEQ ID NO: 6 |
| **P3-3-GS-PEG-Chol** | VKFGDISGINASVVNIKEEIDRLYEVVKNLNESLIDLQEL**GSGSG**C(CH₂CON H(PEG)₄CH₂COOChol)-NH₂, wherein the polypeptide sequence is SEQ ID NO: 7 |

The pseudovirus packages of the SARS-CoV-2 prototype and variants were prepared in-house (see Example 2).

### Example 1

### Pseudovirus packaging of SARS-CoV-2 prototype and variants

### Preparation of expression plasmids for truncated S proteins

1) The nucleotides encoding the last 18 amino acids of S proteins of SARS-CoV-2 prototype and variants (Alpha (B.1.1.7), Beta (B.1.351), Gamma (P.1), Delta (B.1.617.2), and Omicron (BA.1)) were removed to given nucleotide sequences SARS-CoV-2-Prototype-S-del18, B.1.1.7-S-del18, B.1.351-S-del18, P.1-S-del18, B.1.617.2-S-del18, and BA.1-S-del18 (synthesized by Suzhou GENEWIZ), respectively, synthesized by Suzhou GENEWIZ.
2) The nucleotide sequences obtained in 1) were cloned into expression vector pCAGGS to give expression plasmids pCAGGS-SARS-CoV-2-Prototype-S-del18, pCAGGS-B.1.1.7-S-del18, pCAGGS-B.1.351-S-del18, pCAGGS-P.1-S-del18, pCAGGS-B.1.617.2-S-del18, and pCAGGS-BA.1-S-del18, respectively.

### Pseudovirus packaging of SARS-CoV-2 prototype and variants

a. Cell preparation: HEK293T cells were plated on 10-cm cell petri dishes and cultured overnight until a confluence of about 80% was reached.
b. Transfection: The cells in the petri dishes were transfected with the expression plasmids of the S proteins obtained in step 2) at 30 µg of plasmids/10-cm dish by using PEI. The target plasmids and PEI were mixed in a ratio of 1:3, and the cells were transfected. After 4-6 h, the culture medium (DMEM medium containing 10% of FBS) was refreshed before the cells were cultured for 24 h at 37 °C.
c. Virus addition: The pseudovirus packaging virus G*VSV-delG (purchased from BrainVTA (Wuhan) Co., Ltd) was added to the transfected HEK293T cells, and incubated at 37 °C for 2 h. The culture medium (DMEM medium containing 10% FBS) was refreshed, and a VSV-G antibody (expressed by hybridoma cells purchased from ATCC) was added. The system was cultured in an incubator for 30 h.
d. Virus collection: The supernatant was collected, centrifuged at 3000 rpm for 10 min, and filtered through a 0.45-µm sterile filter in an ultra-clean bench. The cell debris was collected, aliquoted, and cryopreserved at -80 °C in a freezer.

After the above procedures, the pseudoviruses of the SARS-CoV-2 prototype and variants (Alpha (B.1.1.7), Beta (B.1.351), Gamma (P.1), Delta (B.1.617.2), and Omicron (BA.1)) were obtained.

### Example 2

### Inhibition effect assessment of polypeptides on pseudoviruses of SARS-CoV-2 prototype and variant

This example was conducted to determine the inhibitory effects of the polypeptides in Table 1 on pseudoviruses of the SARS-CoV-2 prototype and variant Omicron (BA.1).

Grouping: HEK293T ACE2 cells (blank control group, uninfected cells); HEK293T ACE2 cells + prototype or variant pseudovirus + DMEM medium (negative control group, infected cells with no polypeptide treatment); HEK293T ACE2 cells + prototype or variant pseudovirus + polypeptide P3 (P3 treatment group); HEK293T ACE2 cells + prototype or variant pseudovirus + P3-1 (P3-1 treatment group); HEK293T ACE2 cells + prototype or variant pseudovirus + P3-2 (P3-2 treatment group); HEK293T ACE2 cells + prototype or variant pseudovirus + P3-3 (P3-3 treatment group); HEK293T ACE2 cells + prototype or variant pseudovirus + P3-1-GS-Chol (P3-1-GS-Chol treatment group); HEK293T ACE2 cells + prototype or variant pseudovirus + P3-2-GS-Chol (P3-2-GS-Chol treatment group); HEK293T ACE2 cells + prototype or variant pseudovirus + P3-3-GS-Chol (P3-3-GS-Chol treatment group); HEK293T ACE2 cells + prototype or variant pseudovirus + P3-3-GS2-Chol (P3-3-GS2-Chol treatment group); HEK293T ACE2 cells + prototype or variant pseudovirus + P3-1-PEG-Chol (P3-1-PEG-Chol treatment group); HEK293T ACE2 cells + prototype or variant pseudovirus + P3-2-PEG-Chol (P3-2-PEG-Chol treatment group); HEK293T ACE2 cells + prototype or variant pseudovirus + P3-3-PEG-Chol (P3-3-PEG-Chol treatment group); HEK293T ACE2 cells + prototype or variant pseudovirus + P3-1-GS-PEG-Chol (P3-1-GS-PEG-Chol treatment group); HEK293T ACE2 cells + prototype or variant pseudovirus + P3-2-GS-PEG-Chol (P3-2-GS-PEG-Chol treatment group); HEK293T ACE2 cells + prototype or variant pseudovirus + P3-3-GS-PEG-Chol (P3-3-GS-PEG-Chol treatment group).

Preparation of polypeptide stock solutions: The polypeptides in Table 1 were prepared into 10 mM solutions in DMSO, and then diluted to 20 µM in DMEM medium containing 10% of FBS as stock solutions for further serial dilutions.

Preparation of polypeptide serial dilutions: 20 µM of the above polypeptide stock solution was serially 2-fold diluted in DMEM medium containing 10% of FBS to 9 gradients (10 µM, 5 µM, 2.5 µM, 1.25 µM, 0.625 µM, 0.3125 µM, 0.156 µM, 0.078 µM, and 0.039 µM, respectively) at 50 µL/well in triplicate.

Determination of pseudovirus amount: The SARS-CoV-2 prototype or variant pseudovirus stock solutions and serial dilutions were quantified on HEK293T ACE2 cells. The dilution where 1000 FFUs were observed was used as the virus amount (a dilution factor between 6-20 folds) for assessing the polypeptide inhibitory effect.

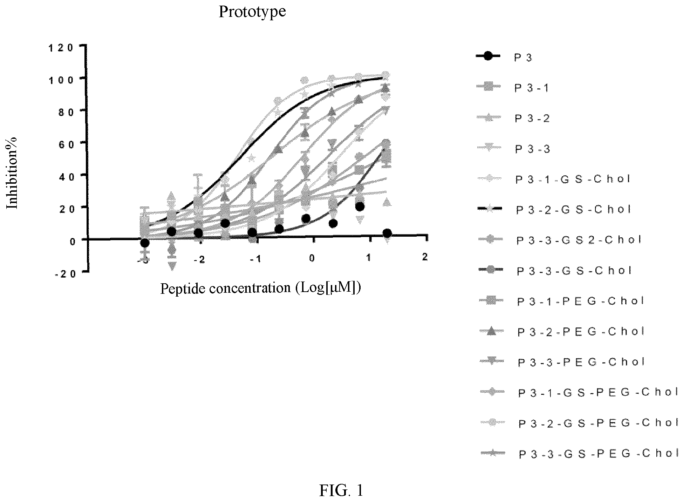

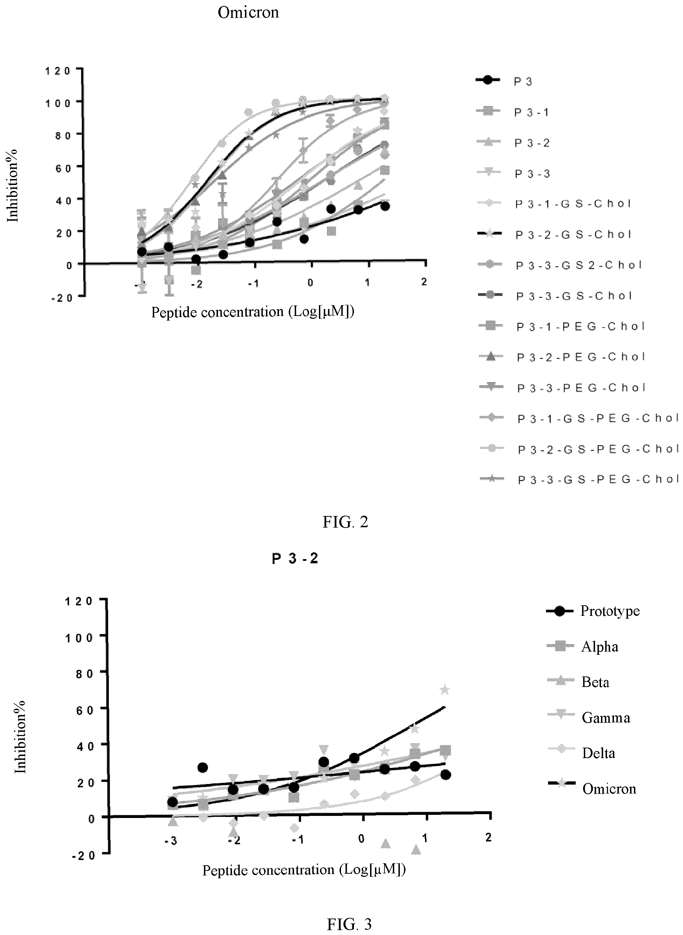

The procedures for assessing the virus inhibitory effect are as follows:
a. HEK293T ACE2 cells were plated in 96-well cell culture plates and cultured overnight until a cell confluence of 80%-90% was reached.
b. According to the grouping, the polypeptide serial dilutions were added along with an equal volume (50 µL) of the pseudovirus dilution, and the systems were mixed well and incubated at 37 °C for 1 h.
c. The supernatant in the 96-well cell culture plates in step a was carefully discarded, before the polypeptide-pseudovirus mixture (100 µL/well) from step b was added. The cells were incubated in an incubator for 15-24 h.
d. The infected cells were counted under a high content microscope, and the inhibitory rates of the polypeptides at different concentrations were calculated. The EC₅₀ values (shown in FIGs. 1 and 2) of the polypeptides were determined by using GraphPad. The assessment was conducted in duplicate to give results designated as 1st and 2nd, as shown in Table 2.

**Table 2. Inhibition effect (EC₅₀) of polypeptides on pseudoviruses of SARS-CoV-2 prototype and variant**

| **EC₅₀(µM)** | **Prototype 1st** | **Omicron 1st** | **Prototype 2nd** | **Omicron 2nd** |
|---|---|---|---|---|
| P3 | 0.3665 | 8.896 | N.D. | N.D. |
| P3-1 | N.D. | 7.899 | N.D. | N.D. |
| P3-2 | 0.1904 | 6.59 | N.D. | 7.289 |
| P3-3 | 0.5011 | 22.66 | N.D. | N.D. |
| P3-1-GS-Chol | 0.03368 | 8.614 | 3.067 | 0.6463 |
| P3-2-GS-Chol | 0.0929 | 0.02208 | 0.05333 | 0.01469 |
| P3-3-GS2-Chol | 0.1991 | 0.6057 | 8.955 | 1.694 |
| P3-3-GS-Chol | 0.1615 | N.D. | 15.67 | 1.729 |
| P3-1-PEG-Chol | 0.1903 | N.D. | 24.02 | 0.959 |
| P3-2-PEG-Chol | 0.2113 | 0.004168 | 0.1955 | 0.01631 |
| P3-3-PEG-Chol | 0.1946 | N.D. | 1.916 | 0.5712 |
| P3-1-GS-PEG-Chol | 0.668 | 0.2818 | 0.6805 | 0.25 |
| P3-2-GS-PEG-Chol | 0.1535 | 0.007021 | 0.0454 | 0.007624 |
| P3-3-GS-PEG-Chol | 0.2596 | 0.1397 | 0.1758 | 0.0198 |

| | | | | |
|---|---|---|---|---|
| Note: N.D. denotes that the calculated half maximal effective concentration (EC₅₀) of the polypeptide for inhibiting the viral strain did not fall within the designed concentration range in this study. | | | | |

### Results and discussion:

As can be seen from Table 2, all of the polypeptides and derivatives thereof exhibited an inhibitory effect against SARS-CoV-2. P3-2 exhibited superior inhibitory effects against the prototype and variant Omicron to those of its original polypeptide P3, but poor repeatability in the inhibitory effect against the prototype.

P3-1-GS-Chol, P3-2-GS-Chol, and P3-3-GS2-Chol exhibited superior inhibitory effects against the prototype and variant Omicron to those of their unmodified counterparts P3-1, P3-2, and P3-3, respectively. P3-2-GS-Chol demonstrated greatly improved inhibitory effects, with the EC₅₀ values in the two duplicate tests being 0.0929 µM and 0.05333 µM against the prototype, and 0.02208 µM and 0.01469 µM against variant Omicron.

P3-1-PEG-Chol, P3-2-PEG-Chol, and P3-3-PEG-Chol exhibited superior inhibitory effects against the prototype and variant Omicron to those of their unmodified counterparts P3-1, P3-2, and P3-3, respectively. P3-2-PEG-Chol demonstrated greatly improved inhibitory effects, with the EC₅₀ values in the two duplicate tests being 0.2113 µM and 0.1955 µM against the prototype, and 0.004168 µM and 0.01631 µM against variant Omicron.

P3-1-GS-PEG-Chol, P3-2-GS-PEG-Chol, and P3-3-GS-PEG-Chol exhibited superior inhibitory effects against the prototype and variant Omicron to those of their unmodified counterparts P3-1, P3-2, and P3-3, respectively. P3-2-GS-PEG-Chol demonstrated greatly improved inhibitory effects, with the EC₅₀ values in the two duplicate tests being 0.1535 µM and 0.0454 µM against the prototype, and 0.007021 µM and 0.007624 µM against variant Omicron.

P3-2-GS-Chol, P3-2-PEG-Chol, and P3-2-GS-PEG-Chol exhibited the best inhibitory effect against the prototype and variant Omicron, especially against variant Omicron. The P3-2 derivatives showed good repeatability in the two duplicate tests, suggesting the most stable inhibitory capability against the viruses.

### Example 3

### Inhibition effect assessment of polypeptide P3-2 and derivatives thereof on pseudoviruses of SARS-CoV-2 prototype and variants

This example was conducted to determine the inhibitory effects of polypeptides P3-2, P3-2-GS-Chol, P3-2-PEG-Chol, P3-2-GS-PEG-Chol, and P3-1-GS-PEG-Chol on pseudoviruses of the SARS-CoV-2 prototype and variants (Alpha (B.1.1.7), Beta (B.1.351), Gamma (P.1), Delta (B.1.617.2), and Omicron (BA.1)).

Grouping: HEK293T ACE2 cells (blank control group, uninfected cells); HEK293T ACE2 cells + prototype or variant pseudovirus + DMEM medium (negative control group, infected cells with no polypeptide treatment); HEK293T ACE2 cells + prototype or variant pseudovirus + polypeptide P3-2 (P3-2 treatment group); HEK293T ACE2 cells + prototype or variant pseudovirus + P3-2-GS-Chol (P3-2-GS-Chol treatment group); HEK293T ACE2 cells + prototype or variant pseudovirus + P3-2-PEG-Chol (P3-2-PEG-Chol treatment group); HEK293T ACE2 cells + prototype or variant pseudovirus + P3-2-GS-PEG-Chol (P3-2-GS-PEG-Chol treatment group); HEK293T ACE2 cells + prototype or variant pseudovirus + P3-1-GS-PEG-Chol (P3-1-GS-PEG-Chol treatment group).

Preparation of polypeptide stock solutions: The polypeptides in Table 1 were prepared into 10 mM solutions in DMSO, and then diluted to 20 µM in DMEM medium containing 10% of FBS as stock solutions for further serial dilutions.

Preparation of polypeptide serial dilutions: 20 µM of the above polypeptide stock solution was serially 2-fold diluted in DMEM medium containing 10% of FBS to 9 gradients (10 µM, 5 µM, 2.5 µM, 1.25 µM, 0.625 µM, 0.3125 µM, 0.156 µM, 0.078 µM, and 0.039 µM, respectively) at 50 µL/well in triplicate.

Determination of pseudovirus amount: The SARS-CoV-2 prototype or variant pseudovirus stock solutions and serial dilutions were quantified on HEK293T ACE2 cells. The dilution where 1000 FFUs were observed was used as the virus amount (a dilution factor between 6-20 folds) for assessing the polypeptide inhibitory effect.

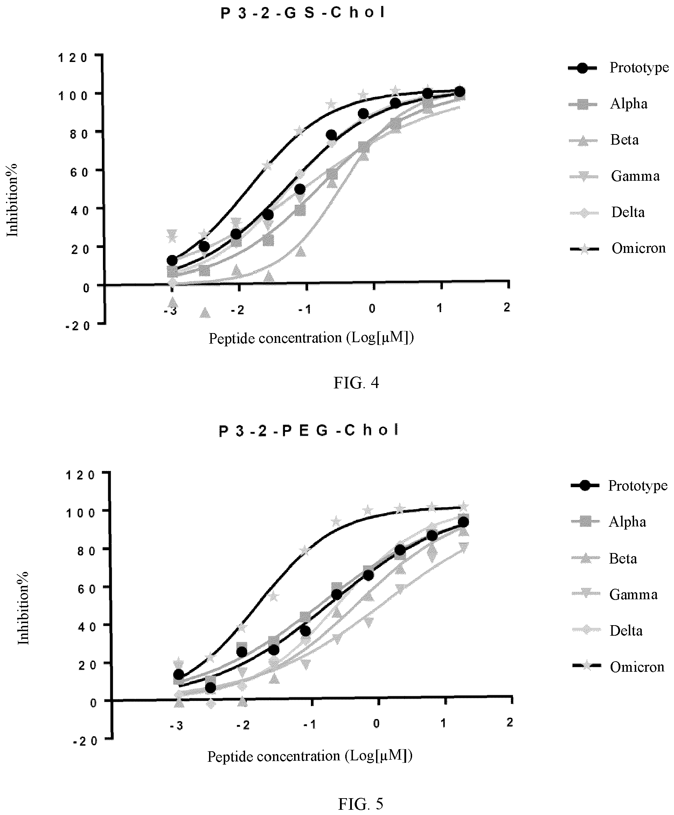

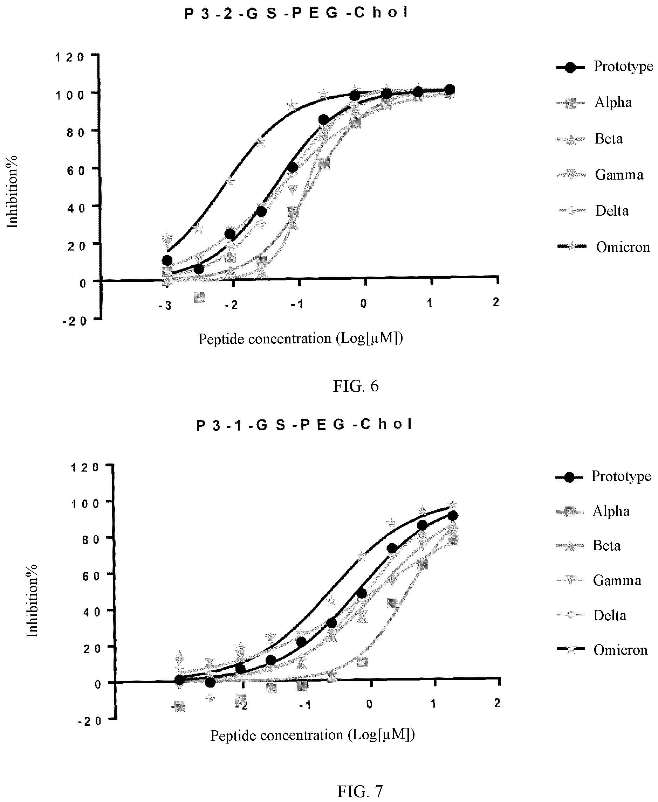

The procedures for assessing the virus inhibitory effect are as follows:
a. HEK293T ACE2 cells were plated in 96-well cell culture plates and cultured overnight until a cell confluence of 80%-90% was reached.
b. According to the grouping, the polypeptide serial dilutions were added along with an equal volume (50 µL) of the pseudovirus dilution, and the systems were mixed well and incubated at 37 °C for 1 h.
c. The supernatant in the 96-well cell culture plates in step a was carefully discarded, before the polypeptide-pseudovirus mixture (100 µL/well) from step b was added. The cells were incubated in an incubator for 15-24 h.
d. The infected cells were counted under a high content microscope, and the inhibitory rates of the polypeptides at different concentrations were calculated. The EC₅₀ values (shown in FIGs. 3-7) of the polypeptides were determined by using GraphPad. The results are shown in Table 3.

**Table 3. Inhibition effect (EC₅₀) of polypeptide P3-2 and derivatives thereof on pseudoviruses of SARS-CoV-2 prototype and variants**

| EC₅₀(µM) | Prototype | Alpha | Beta | Gamma | Delta | Omicron |
|---|---|---|---|---|---|---|
| P3-2 | N.D. | 327.9 | N.D. | 1304 | 280.7 | 7.289 |
| P3-2-GS-Chol | 0.05333 | 0.1618 | 0.3385 | 0.09824 | 0.0586 | 0.01469 |
| P3-2-PEG-Chol | 0.1955 | 0.1443 | 0.5096 | 1.257 | 0.2623 | 0.01631 |
| P3-2-GS-PEG-Chol | 0.0454 | 0.1605 | 0.1359 | 0.05655 | 0.05991 | 0.007624 |
| P3-1-GS-PEG-Chol | 0.6805 | 4.093 | 1.373 | 1.285 | 0.9383 | 0.25 |

| | | | | | | |
|---|---|---|---|---|---|---|
| **Note: N.D. denotes** that the calculated half maximal effective concentration (EC₅₀) of the polypeptide for inhibiting the viral strain did not fall within the designed concentration range in this study. | | | | | | |

### Results and discussion

As seen from Table 3, the modified derivatives P3-2-GS-Chol, P3-2-PEG-Chol, and P3-2-GS-PEG-Chol of polypeptide P3-2 not only exhibited good inhibitory effects against the prototype and variant Omicron of SARS-CoV-2, but also demonstrated good inhibitory effects against the other variants Alpha, Beta, Gamma, and Delta, as well as good repeatability in inhibition test results against the prototype and variant Omicron. Compared with the derivative P3-1-GS-PEG-Chol of polypeptide P3-1, the derivatives P3-2-GS-Chol, P3-2-PEG-Chol, and P3-2-GS-PEG-Chol of polypeptide P3-2 exhibited better inhibitory effects against the prototype and variants Alpha, Beta, Gamma, Delta, and Omicron.

Compared with P3-2, P3-2-GS-Chol, P3-2-PEG-Chol, and P3-2-GS-PEG-Chol exhibited better inhibitory effects against the prototype and variants Alpha, Beta, Gamma, Delta, and Omicron, and the inhibitory effects of P3-2-GS-PEG-Chol were superior to those of P3-2-GS-Chol and P3-2-PEG-Chol.

Among variants Alpha, Beta, Gamma, Delta, and Omicron, P3-2-GS-Chol, P3-2-PEG-Chol, and P3-2-GS-PEG-Chol demonstrated the best inhibitory effect against Omicron.

### Example 4

### Inhibition effect assessment of truncated polypeptides of P3-2-GS-PEG-Chol and P3-2-GS-Chol on pseudoviruses of SARS-CoV-2 prototype and variants

This example was conducted to determine the inhibitory effects of truncated polypeptides obtained by continuous single amino acid deletions at the N-terminus of polypeptides P3-2-GS-Chol and P3-2-GS-PEG-Chol on pseudoviruses of the SARS-CoV-2 prototype and variants (Delta (B. 1.617.2) and Omicron (BA. 1)).

The amino acid sequences of the truncated polypeptides obtained by continuous single amino acid deletions at the N-terminus of polypeptides P3-2-GS-Chol and P3-2-GS-PEG-Chol are shown in Tables 4 and 5.

**Table 4. Amino acid sequence of truncated polypeptides obtained by continuous single amino acid deletions at N-terminus of polypeptide P3-2-GS-Chol**

| Name | Sequence |
|---|---|
| **P3-2** | VDLGDISGINASVVNIQKEIDRLNEVAKNLNESLIDLQEL SEQ ID NO.2 |
| **P3-2-1** | DLGDISGINASVVNIQKEIDRLNEVAKNLNESLIDLQEL SEQ ID NO.12 |
| **P3-2-2** | LGDISGINASVVNIQKEIDRLNEVAKNLNESLIDLQEL SEQ ID NO.13 |
| **P3-2-3** | GDISGINASVVNIQKEIDRLNEVAKNLNESLIDLQEL SEQ ID NO.14 |
| **P3-2-GS-Chol** | VDLGDISGINASVVNIQKEIDRLNEVAKNLNESLIDLQELGSGSGC(CH₂CO OChol)-NH₂ SEQ ID NO.6 |
| **P3-2-GS-Chol-1** | DLGDISGINASVVNIQKEIDRLNEVAKNLNESLIDLQELGSGSGC(CH₂COO Chol)-NH₂ SEQ ID NO.15 |
| **P3-2-GS-Chol-2** | LGDISGINASVVNIQKEIDRLNEVAKNLNESLIDLQELGSGSGC(CH2COOC hol)-NH2 SEQ ID NO.16 |
| **P3-2-GS-Chol-3** | GDISGINASVVNIQKEIDRLNEVAKNLNESLIDLQELGSGSGC(CH2COOCh ol)-NH2 SEQ ID NO.17 |

**Table 5. Amino acid sequence of truncated polypeptides obtained by continuous single amino acid deletions at N-terminus of polypeptide P3-2-GS-PEG-Chol**

| **Name** | **Sequence** |
|---|---|
| **P3-2-GS-PEG-Chol** | VDLGDISGINASVVNIQKEIDRLNEVAKNLNESLIDLQELGSGSGC(CH2 CONH(PEG)4CH2COOChol)-NH2 SEQ ID NO.6 |
| **P3-2-GS-PEG-Chol -1** | DLGDISGINASVVNIQKEIDRLNEVAKNLNESLIDLQELGSGSGC(CH2C ONH(PEG)4CH2COOChol)-NH2 SEQ ID NO.15 |
| **P3-2-GS-PEG-Chol -2** | LGDISGINASVVNIQKEIDRLNEVAKNLNESLIDLQELGSGSGC(CH₂CO NH(PEG)₄CH₂COOChol)-NH₂ SEQ ID NO.16 |
| **P3-2-GS-PEG-Chol -3** | GDISGINASVVNIQKEIDRLNEVAKNLNESLIDLQELGSGSGC(CH2CON H(PEG)4CH2COOChol)-NH2 SEQ ID NO.17 |

Since P3-2-GS-Chol-1 and P3-2-GS-PEG-Chol-1 were extremely unstable during the purification process of synthesis, they were not subjected to further inhibitory effect assessment on the pseudoviruses of SARS-CoV-2 prototype and variants.

Grouping: HEK293T ACE2 cells (blank control group, uninfected cells); HEK293T ACE2 cells + prototype or variant pseudovirus + DMEM medium (negative control group, infected cells with no polypeptide treatment); HEK 293T ACE2 cells + prototype or variant pseudovirus + P3-2-GS-PEG-Chol (P3-2-GS-PEG-Chol treatment group); HEK 293T ACE2 cells + prototype or variant pseudovirus + polypeptide P3-2-GS-PEG-Chol-2 (P3-2-GS-PEG-Chol-2 treatment group); HEK 293T ACE2 cells + prototype or variant pseudovirus + P3-2-GS-PEG-Chol-3 (P3-2-GS-PEG-Chol-3 treatment group); HEK 293T ACE2 cells + prototype or variant pseudovirus + P3-2-GS-Chol-2 (P3-2-GS-Chol-2 treatment group); HEK 293T ACE2 cells + prototype or variant pseudovirus + P3-2-GS-Chol-3 (P3-2-GS-Chol-3 treatment group).

Preparation of polypeptide stock solutions: The polypeptides in Tables 4 and 5 were prepared into 10 mM solutions in DMSO, and then diluted to 20 µM in DMEM medium containing 10% of FBS as stock solutions for further serial dilutions.

Preparation of polypeptide serial dilutions: 20 µM of the above polypeptide stock solution was serially 3-fold diluted in DMEM medium containing 10% of FBS to 9 gradients (6.67, 2.22, 0.74, 0.25, 0.082, 0.027, 0.009, 0.003, and 0.001 µM, respectively) at 50 µL/well in triplicate.

Determination of pseudovirus amount: The SARS-CoV-2 prototype or variant pseudovirus stock solutions and serial dilutions were quantified on HEK293T ACE2 cells. The dilution where 1000 FFUs were observed was used as the virus amount (a dilution factor between 6-20 folds) for assessing the polypeptide inhibitory effect.

The procedures for assessing the virus inhibitory effect are as follows:
a. HEK293T ACE2 cells were plated in 96-well cell culture plates and cultured overnight until a cell confluence of 80%-90% was reached.
b. According to the grouping, the polypeptide serial dilutions were added along with an equal volume (50 µL) of the pseudovirus dilution, and the systems were mixed well and incubated at 37 °C for 1 h.
c. The supernatant in the 96-well cell culture plates in step a was carefully discarded, before the polypeptide-pseudovirus mixture (100 µL/well) from step b was added. The cells were incubated in an incubator for 15-24 h.
d. The infected cells were counted under a high content microscope, and the inhibitory rates of the polypeptides at different concentrations were calculated. The EC₅₀ values of the polypeptides were determined by using GraphPad. The results are shown in Table 6.

**Table 6. Inhibition effect (EC₅₀) of truncated polypeptides of P3-2-GS-PEG-Chol and P3-2-GS-Chol on pseudoviruses of SARS-CoV-2 prototype and variants**

| **EC₅₀ (µM)** | **Prototype** | **Delta** | **Omicron** |
|---|---|---|---|
| P3-2-GS-PEG-Chol | 0.03110 | 0.01589 | 0.01085 |
| P3-2-GS-PEG-Chol-2 | 0.2100 | 0.02260 | 0.01156 |
| P3-2-GS-PEG-Chol-3 | 1.585 | 0.04737 | 0.1563 |
| P3-2-GS-Chol-2 | 0.8645 | 1.248 | 0.1928 |
| P3-2-GS-Chol-3 | 0.7789 | 0.6295 | 0.06472 |

### Results and discussion

In this example, the truncated polypeptides P3-2-GS-Chol-1 and P3-2-GS-PEG-Chol-1 were prepared from P3-2-GS-Chol and P3-2-GS-PEG-Chol by solid-phase synthesis. However, the two polypeptides exhibited poor stability, and cannot be purified to give a high-purity sample. Therefore, the two polypeptides were not subjected to the pseudovirus neutralization assay for assessing the inhibitory effect against viruses.

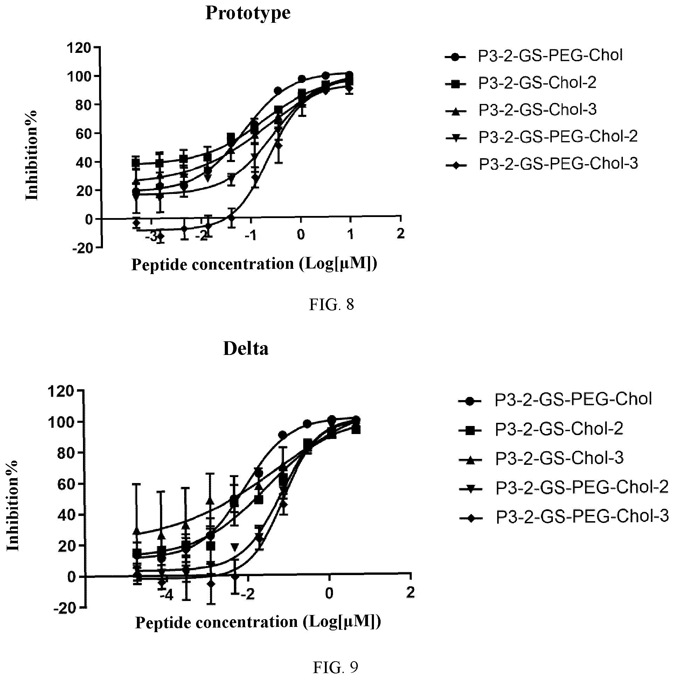

As seen from Table 6 and FIGs. 8-10, the truncated polypeptides P3-2-GS-PEG-Chol-2 and P3-2-GS-PEG-Chol-3 of polypeptide P3-2-GS-PEG-Chol exhibited good inhibitory effects against the SARS-CoV-2 prototype and variant Delta and Omicron, but the inhibitory effects were weaker than those of P3-2-GS-PEG-Chol, and such inhibitory effects were not reduced along with the continuous truncation of the peptide sequence; the truncated polypeptides P3-2-GS-Chol-2, P3-2-GS-Chol-3, P3-2-GS-Chol-4, and P3-2-GS-Chol-5 of polypeptide P3-2-GS-Chol also exhibited certain inhibitory effects against the prototype and variants Delta and Omicron, but the inhibitory effects were weaker than those of the P-2-GS-Chol.

Comparing the pseudovirus neutralization assay data of the truncated polypeptides and the original polypeptides, the antiviral effect of the truncated polypeptide of P3-2-GS-Chol and P3-2-GS-PEG-Chol showed reductions to different extents.

### Example 5

Inhibitory effect assessment of polypeptide P3-2 derivatives on live SARS-CoV-2 prototype and variants in Vero cells

This example was conducted to determine the inhibitory effects of polypeptides P3-2-GS-Chol, P3-2-PEG-Chol, and P3-2-GS-PEG-Chol in Table 1 on live SARS-CoV-2 prototype (SARS-CoV-2 WH01) and variants (Delta (B.1.617.2) and Omicron (BA. 1)) in Vero cells. Grouping: Vero cells (blank control group, uninfected cells); Vero cells + live prototype or variant virus + DMEM medium (negative control group, infected cells with no polypeptide treatment); Vero cells + live prototype or variant virus + P3-2-GS-Chol (P3-2-GS-Chol treatment group); Vero cells + live prototype or variant virus + P3-2-PEG-Chol (P3-2-PEG-Chol treatment group); Vero cells + live prototype or variant virus + P3-2-GS-PEG-Chol (P3-2-GS-PEG-Chol treatment group).

Preparation of polypeptide stock solutions: The polypeptides P3-2, P3-2-GS-Chol, P3-2-PEG-Chol, and P3-2-GS-PEG-Chol in Table 1 were prepared into 5 mM solutions in DMSO, and then diluted to 5 µM in DMEM medium containing 2% of FBS as stock solutions for further serial dilutions. Polypeptides P3-2-GS-Chol and P3-2-PEG-Chol were serially 3-fold diluted from 5 µM to 11 concentrations for the three virus strains; polypeptide P3-2-GS-PEG-Chol were serially 3-fold diluted from 5 µM for the SARS-CoV-2 prototype and variant Delta and 5-fold from 5 µM for variant Omicron to 11 concentrations; each concentration was prepared in 4 replicates at 50 µL/well. Determination of live virus amount: Based on TCID50, virus solutions of SARS-CoV-2 WH01, Delta, and Omicron were diluted to 2000 TCID50/mL, each requiring 9.6 mL (prepared at 12 mL). DMEM (containing 2% of FBS) was used for dilution.

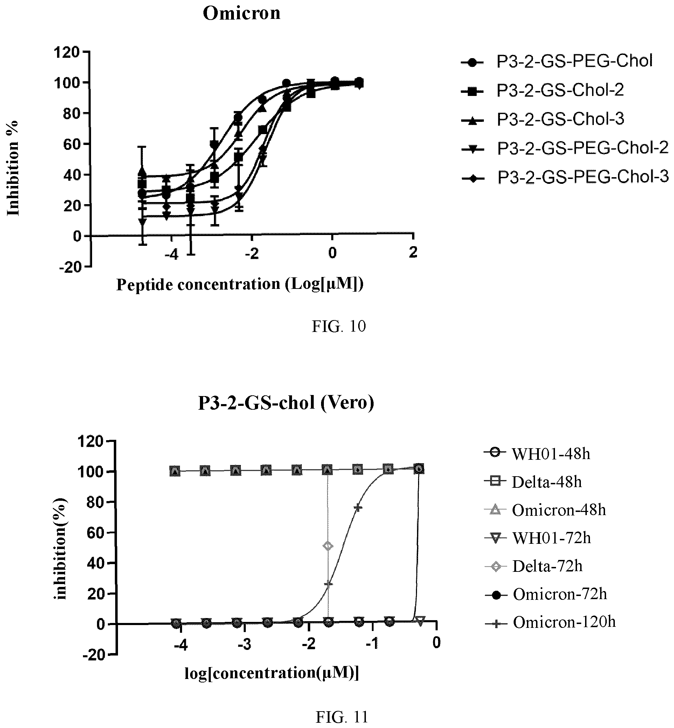

The procedures for assessing the virus inhibitory effect are as follows:
a. According to the grouping, the polypeptide serial dilutions were added along with an equal volume (50 µL) of the live virus dilution, and the systems were mixed well and incubated at 37 °C for 1 h.
b. Suspended Vero cells were added to the mixture, and the system was incubated for 48-120 h in an incubator.
c. The cytopathic conditions were observed, and the number of cytopathic wells was recorded at 48 h, 72 h and 120 h for calculating the inhibition rate of the polypeptides at different concentrations. The EC₅₀ results (shown in FIGs. 11-13) of the polypeptides were calculated by utilizing GraphPad, as shown in Table 7.

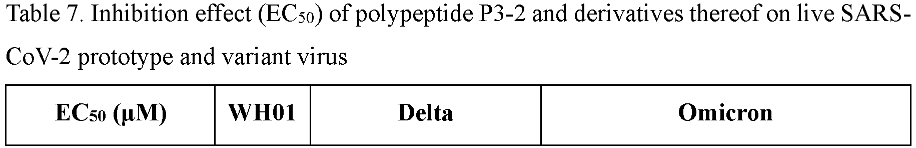

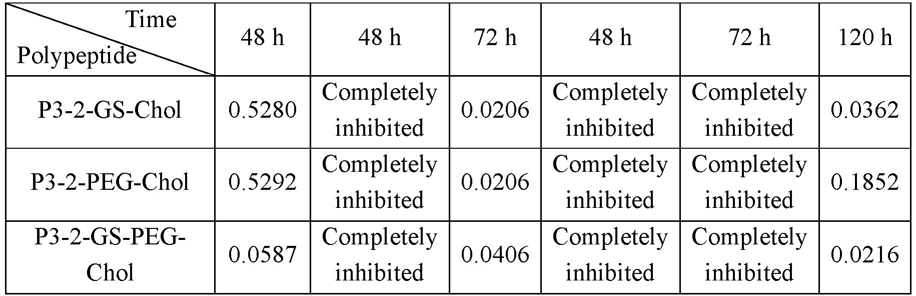

### Results and discussion

As can be seen from Table 7, P3-2-GS-Chol, P3-2-PEG-Chol, and P3-2-GS-PEG-Chol exhibited excellent inhibitory effects against live SARS-CoV-2 viruses in Vero cells.

In the live virus neutralization assay for P3-2-GS-Chol, according to the CPE (cytopathic effect) observed at 48 hours, the calculated EC₅₀ for the SARS-CoV-2 prototype (WH01) was 0.5280 µM. For SARS-CoV-2 variant Delta, no CPE was observed in the treatment group at 48 hours, where the virus inhibition rate for the polypeptide was 100%; at 72 hours, CPE was observed, and the calculated EC₅₀ was 0.0206 µM. For SARS-CoV-2 variant Omicron, no CPE was observed in the treatment group at 48 or 72 hours, where the virus inhibition rate for the polypeptide was 100%; at 120 hours, CPE was observed, and the calculated EC₅₀ was 0.0362 µM.

In the live virus neutralization assay for P3-2-PEG-Chol, according to the CPE observed at 48 hours, the calculated EC₅₀ for the SARS-CoV-2 prototype (WH01) was 0.5292 µM. For SARS-CoV-2 variant Delta, no CPE was observed in the treatment group at 48 hours, where the virus inhibition rate for the polypeptide was 100%; at 72 hours, CPE was observed, and the calculated EC₅₀ was 0.0206 µM. For SARS-CoV-2 variant Omicron, no CPE was observed in the treatment group at 48 or 72 hours, where the virus inhibition rate for the polypeptide was 100%; at 120 hours, CPE was observed, and the calculated EC₅₀ was 0.1852 µM.

In the live virus neutralization assay for P3-2-GS-PEG-Chol, according to the CPE observed at 48 hours, the calculated EC₅₀ for the SARS-CoV-2 prototype (WH01) was 0.0587 µM. For SARS-CoV-2 variant Delta, no CPE was observed in the treatment group at 48 hours, where the virus inhibition rate for the polypeptide was 100%; at 72 hours, CPE was observed, and the calculated EC₅₀ was 0.0406 µM. For SARS-CoV-2 variant Omicron, no CPE was observed in the treatment group at 48 or 72 hours, where the virus inhibition rate for the polypeptide was 100%; at 120 hours, CPE was observed, and the calculated EC₅₀ was 0.0216 µM.

The observation and the EC₅₀ analysis suggest that P3-2-GS-Chol, P3-2-PEG-Chol, and P3-2-GS-PEG-Chol are effective against live SARS-CoV-2 viruses in Vero cells, especially for Omicron.

### Example 6

Inhibitory effect assessment of polypeptide P3-2 derivatives on live SARS-CoV-2 prototype and variants in Vero E6 cells

This example was conducted to determine the inhibitory effects of polypeptides P3-2-GS-Chol, P3-2-PEG-Chol, and P3-2-GS-PEG-Chol in Table 1 on live SARS-CoV-2 prototype (SARS-CoV-2 WH01) and variants (Delta (B.1.617.2) and Omicron (BA.1)) in Vero E6 cells.

Grouping: Vero E6 cells (blank control group, uninfected cells); Vero E6 cells + live prototype or variant virus + DMEM medium (negative control group, infected cells with no polypeptide treatment); Vero E6 cells + live prototype or variant virus + P3-2-GS-Chol (P3-2-GS-Chol treatment group); Vero E6 cells + live prototype or variant virus + P3-2-PEG-Chol (P3-2-PEG-Chol treatment group); Vero E6 cells + live prototype or variant virus + P3-2-GS-PEG-Chol (P3-2-GS-PEG-Chol treatment group).

Preparation of polypeptide stock solutions: The polypeptides P3-2, P3-2-GS-Chol, P3-2-PEG-Chol, and P3-2-GS-PEG-Chol in Table 1 were prepared into 5 mM solutions in DMSO, and then diluted to 5 µM in DMEM medium containing 2% of FBS as stock solutions for further serial dilutions. Polypeptides P3-2-GS-Chol and P3-2-PEG-Chol were serially 3-fold diluted from 5 µM to 11 concentrations for the three virus strains; polypeptide P3-2-GS-PEG-Chol were serially 3-fold diluted from 5 µM for the SARS-CoV-2 prototype and variant Delta and 5-fold from 5 µM for variant Omicron to 11 concentrations; each concentration was prepared in 4 replicates at 50 µL/well. Determination of live virus amount: Based on TCID50, virus solutions of SARS-CoV-2 WH01, Delta, and Omicron were diluted to 2000 TCID50/mL, each requiring 9.6 mL (prepared at 12 mL). DMEM (containing 2% of FBS) was used for dilution.

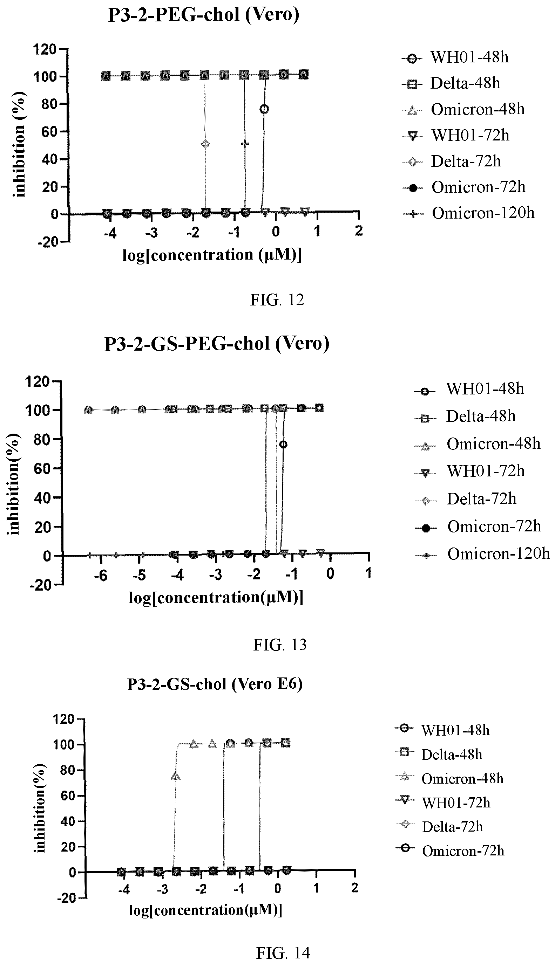

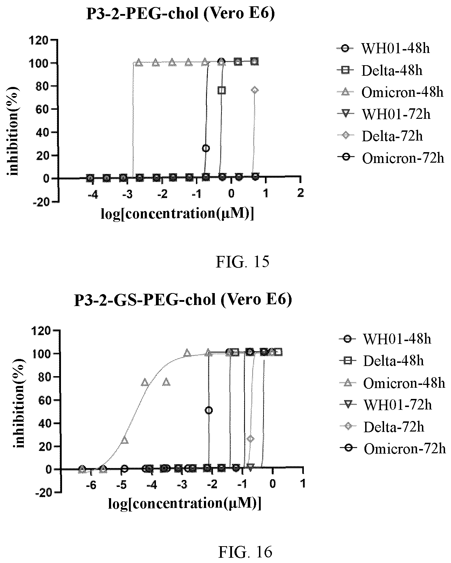

The procedures for assessing the virus inhibitory effect are as follows:
a. According to the grouping, the polypeptide serial dilutions were added along with an equal volume (50 µL) of the live virus dilution, and the systems were mixed well and incubated at 37 °C for 1 h.
b. Suspended Vero E6 cells were added to the mixture, and the system was incubated for 48-72 h in an incubator.
c. The cytopathic conditions were observed, and the number of cytopathic wells was recorded at 48 h and 72 h for calculating the inhibition rate of the polypeptides at different concentrations. The EC₅₀ results (shown in FIGs. 14-16) of the polypeptides were calculated by utilizing GraphPad, as shown in Table 8.

**Table 8. Inhibitory effect (EC₅₀) of polypeptide P3-2 and derivatives thereof on live SARS-CoV-2 prototype and variants in Vero E6 cells at 48 h and 72 h**

| EC₅₀ (µM) | **WH01** | | **Delta** | | **Omicron** | |
|---|---|---|---|---|---|---|
| | 48 h | 72 h | 48 h | 72 h | 48 h | 72 h |
| P3-2-GS-Chol | N.D | N.D | 0.3485 | N.D | 0.0022 | 0.0403 |
| P3-2-PEG-Chol | N.D | N.D | 0.5292 | 4.754 | 0.0015 | 0.1947 |
| P3-2-GS-PEG-Chol | 0.1208 | 0.5280 | 0.0403 | 0.2031 | 0.000028 | 0.008 |

| | | | | | | |
|---|---|---|---|---|---|---|
| **Note: N.D. denotes** that the calculated half maximal effective concentration (EC₅₀) of the polypeptide for inhibiting the viral strain did not fall within the designed concentration range in this study. | | | | | | |

### Results and discussion

As can be seen from Table 8, P3-2-GS-Chol, P3-2-PEG-Chol, and P3-2-GS-PEG-Chol exhibited excellent inhibitory effects against live SARS-CoV-2 viruses in Vero E6 cells. Since the EC₅₀ was observed after 48 h for various polypeptides, the study mainly compared the data at 48 h.

In the live virus neutralization assay for P3-2-GS-Chol, no inhibitory effect against live SARS-CoV-2 prototype virus (WH01) was observed at both 48 h and 72 h in Vero E6 cells. For SARS-CoV-2 variant Delta, CPE was observed in the treatment group at 48 hours, and the calculated EC₅₀ was 0.3485 µM. For SARS-CoV-2 variant Omicron, CPE was observed in the treatment group at 48 hours, and the calculated EC₅₀ was 0.0022 µM.

In the live virus neutralization assay for P3-2-PEG-Chol, no inhibitory effect against live SARS-CoV-2 prototype virus (WH01) was observed at both 48 h and 72 h in Vero E6 cells. For SARS-CoV-2 variant Delta, CPE was observed in the treatment group at 48 hours, and the calculated EC₅₀ was 0.5292 µM. For SARS-CoV-2 variant Omicron, CPE was observed in the treatment group at 48 hours, and the calculated EC₅₀ was 0.0015 µM.

In the live virus neutralization assay for P3-2-GS-PEG-Chol, CPE was observed at 48 hours in the treatment group for SARS-CoV-2 variant Delta in Vero E6 cells, and the calculated EC₅₀ was 0.1208 µM. For SARS-CoV-2 variant Delta, CPE was observed in the treatment group at 48 hours, and the calculated EC₅₀ was 0.0403 µM. For SARS-CoV-2 variant Omicron, CPE was observed in the treatment group at 48 hours, and the calculated EC₅₀ was 0.000028 µM.

The observation and the EC₅₀ analysis suggest that P3-2-GS-Chol and P3-2-PEG-Chol are effective against the live SARS-CoV-2 variants in Vero E6 cells, with the best inhibitory effect observed for Omicron; P3-2-GS-PEG-Chol is effective against the live SARS-CoV-2 prototype and variants, with the best inhibitory effect observed for Omicron.

In conclusion, in the process of SARS-CoV-2 invading host cells, the polypeptides and the derivatives thereof in the present invention can inhibit virus invasion by inhibiting the S protein-mediated membrane fusion process, and are thus suitable for preventing and treating diseases caused by coronaviruses, especially the SARS-CoV-2 prototype and variants. The polypeptide and the derivatives thereof have significant inhibitory effects against the SARS-CoV-2 prototype and variants Alpha, Beta, Gamma, Delta, and Omicron, especially for variant Omicron, and thus have broad application prospects.

The abbreviations and their full versions referred to in Examples 1-6 are shown in Table 9 below.

**Table 9. Abbreviations and full versions**

| Abbreviation | Full version |
|---|---|
| FBS | Fetal bovine serum |
| CPE | Cytopathic effect |
| Chol | Cholesterol |
| PEG | Polyethylene glycol |
| A | Alanine |
| R | Arginine |
| N | Asparagine |
| D | Aspartic acid |
| C | Cysteine |
| Q | Glutamine |
| E | Glutamic acid |
| G | Glycine |
| H | Histidine |
| I | Isoleucine |
| L | Leucine |
| K | Lysine |
| M | Methionine |
| F | Phenylalanine |
| P | Proline |
| S | Serine |
| T | Threonine |
| W | Tryptophan |
| Y | Tyrosine |
| V | Valine |

It is obvious that the above examples are merely illustrative for a clear explanation and are not intended to limit the embodiments. Various changes and modifications can be made by those of ordinary skills in the art on the basis of the above description. It is unnecessary and impossible to exhaustively list all the embodiments herein. Obvious changes or modifications derived therefrom still fall within the protection scope of the present invention.

## Claims

1. An anti-coronavirus polypeptide, wherein the anti-coronavirus polypeptide is a polypeptide having any one of the following sequences (1) to (8):
(1) a polypeptide comprising the amino acid sequence set forth in SEQ ID NO: 1;
(2) a polypeptide, comprising the amino acid sequence set forth in SEQ ID NO: 2;
(3) a polypeptide, comprising the amino acid sequence set forth in SEQ ID NO: 3;
(4) a polypeptide, comprising the amino acid sequence set forth in SEQ ID NO: 12;
(5) a polypeptide, comprising the amino acid sequence set forth in SEQ ID NO: 13;
(6) a polypeptide, comprising the amino acid sequence set forth in SEQ ID NO: 14;
(7) a polypeptide, comprising an amino acid sequence having 1 or more amino acid residue substitutions, deletions, additions, or insertions compared with any one of the amino acid sequences of the polypeptides (1) to (6), and having an inhibitory activity against a coronavirus; or
(8) a polypeptide, comprising an amino acid sequence having at least 60%, 65%, 70%, 75%, 80%, 85%, 90%, 93%, 94%, or 95% identity to any one of the amino acid sequences of the polypeptides (1) to (6), and having an inhibitory activity against a coronavirus; preferably, the sequence of the polypeptide is SEQ ID NO: 1, SEQ ID NO: 2, SEQ ID NO: 3, SEQ ID NO: 12, SEQ ID NO: 13, or SEQ ID NO: 14.

2. An anti-coronavirus polypeptide derivative, wherein the polypeptide derivative is the anti-coronavirus polypeptide according to claim 1 modified by a cholesterol derivative; preferably, the site of the cholesterol derivative modification is at the C-terminus or N-terminus or on a basic amino acid side chain of the polypeptide; more preferably, the site of the cholesterol derivative modification is at the C-terminus of the polypeptide; preferably, the cholesterol derivative is linked to the C-terminus of the polypeptide via a linker or directly;
preferably, the cholesterol derivative is a derivative of cholesterol modified by cysteine; or
the cholesterol derivative is a derivative of cholesterol modified by cysteine and PEG.

3. The polypeptide derivative according to claim 2, wherein,
the cholesterol derivative is selected from 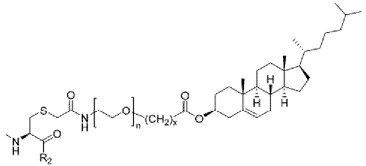
and 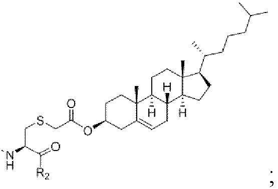
R₂ is OH or NH₂;
n is any integer of 1-20, preferably any integer of 2-6;
x is any integer of 1-6.

4. The polypeptide derivative according to claim 2 or 3, wherein the linker is -NH-PEGn-(CH₂)x-CO-, (GSG)m, (GSGSG)m, (GSG)m-CONH-PEGn-(CH₂)x-CO-, or (GSGSG)m-CONH-PEGn-(CH₂)x-CO-;
m is any integer of 0-6;
n is any integer of 1-20, preferably any integer of 2-6;
x is any integer of 1-6.

5. The polypeptide derivative according to claim 2, wherein the polypeptide derivative has a structural formula represented by the following structure I, structure II, and structure III: 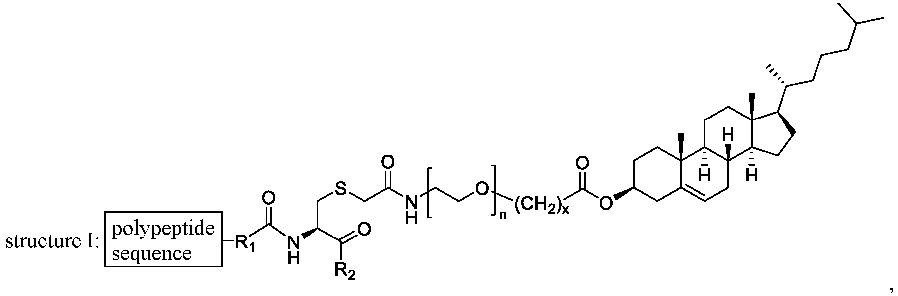 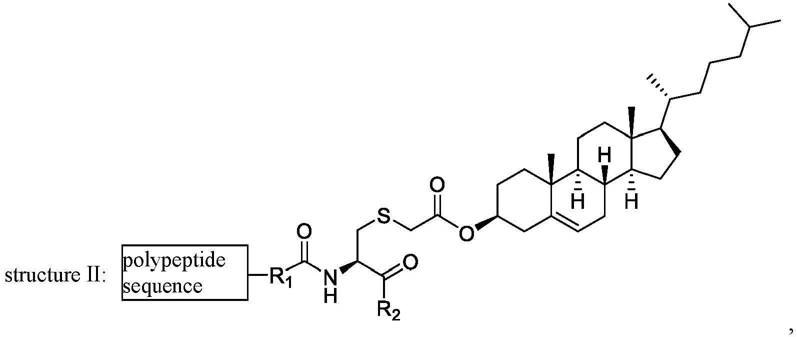 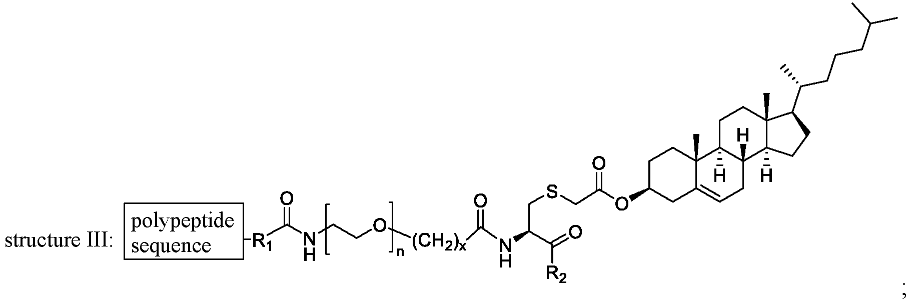
R₁ is (GSG)m or (GSGSG)m; m is any integer of 0-6, and preferably m is 0, 1 or 2;
R₂ is OH or NH₂; preferably, R₂ is NH₂;
the polypeptide sequence is selected from the polypeptide sequence set forth in any one of (1) to (8) in claim 1;
preferably, the polypeptide sequence is SEQ ID NO: 1, SEQ ID NO: 2, SEQ ID NO: 3, SEQ ID NO: 12, SEQ ID NO: 13, or SEQ ID NO: 14.

6. The polypeptide derivative according to claim 2, wherein the polypeptide derivative is selected from:

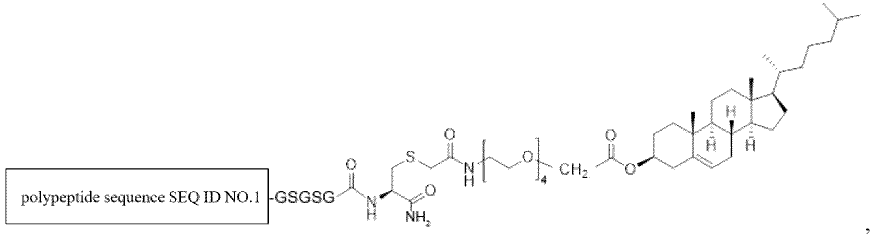

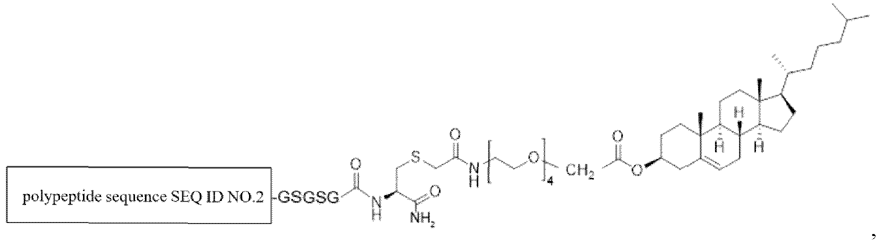

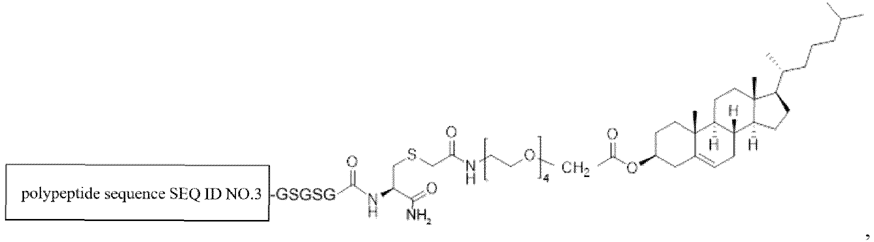

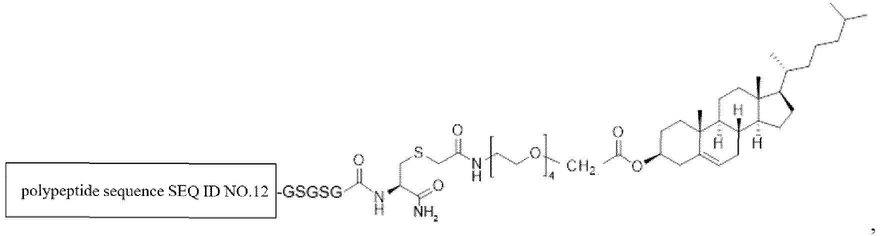

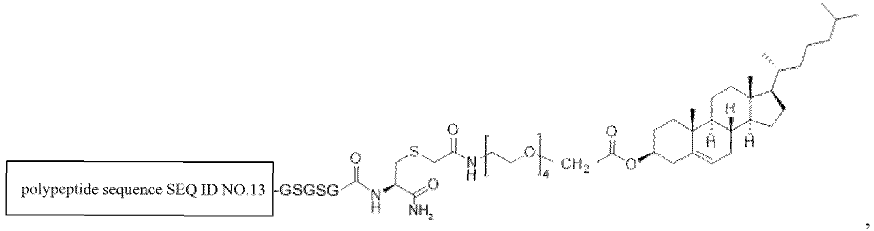

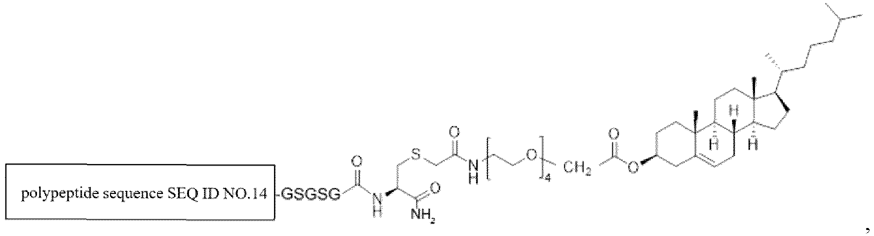

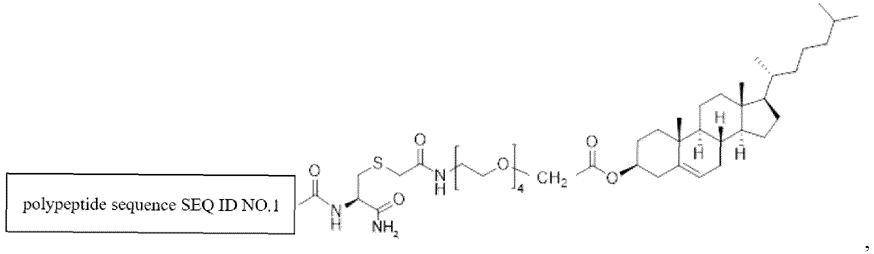

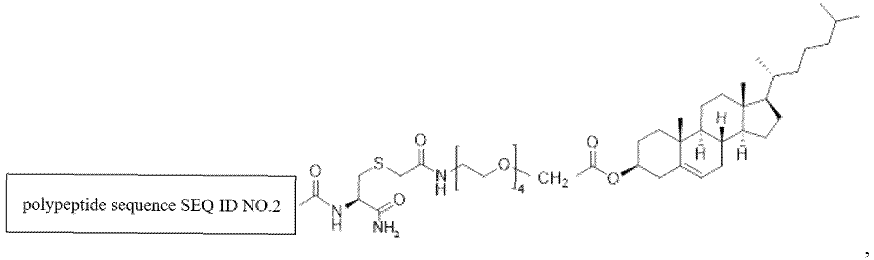

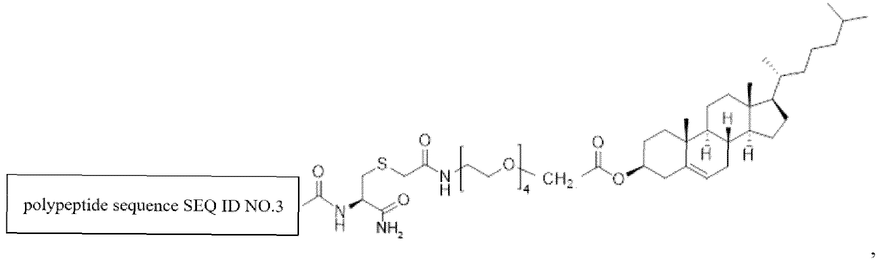

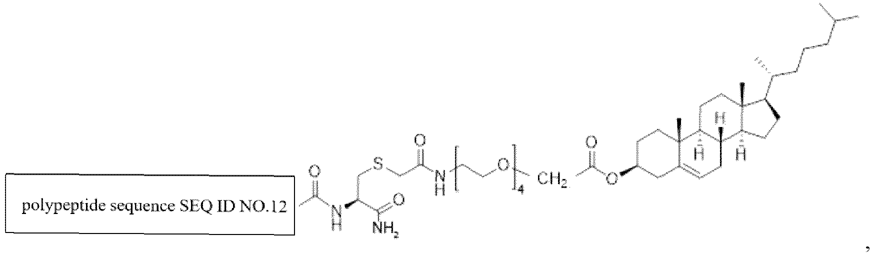

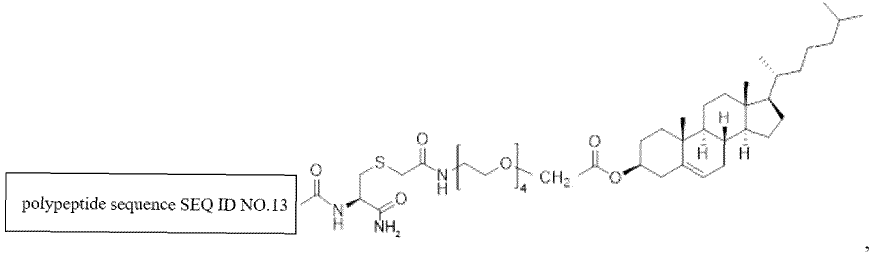

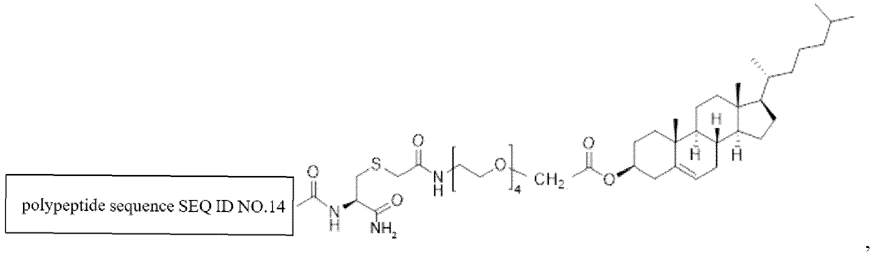

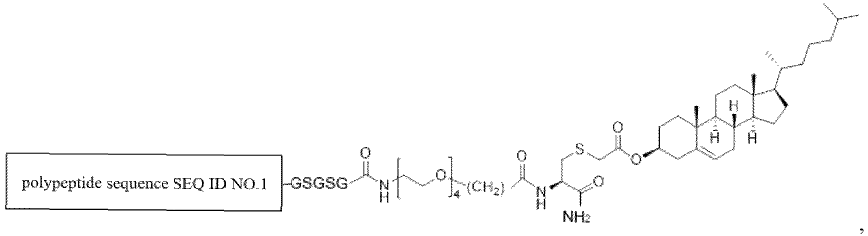

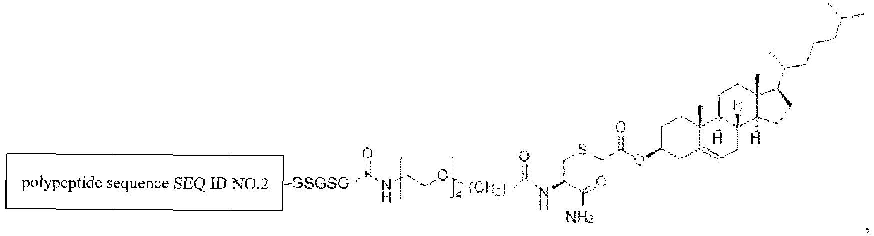

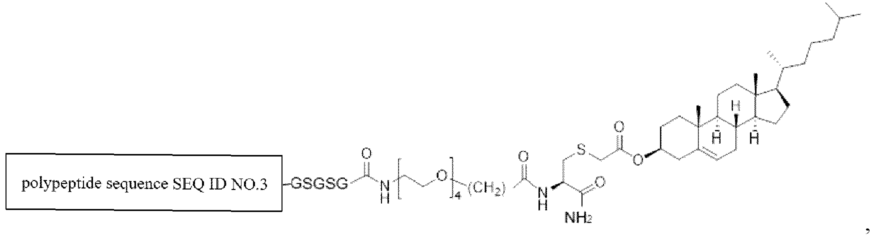

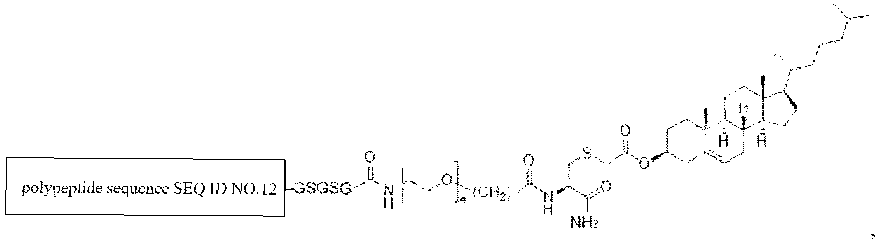

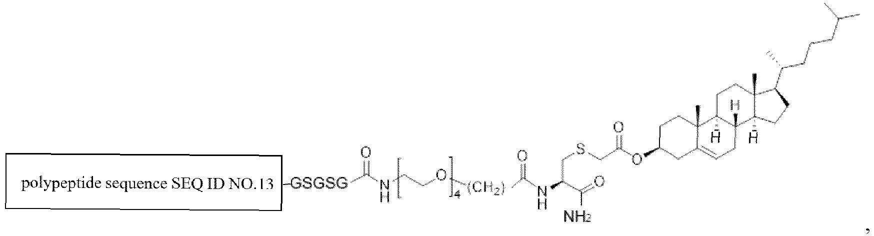

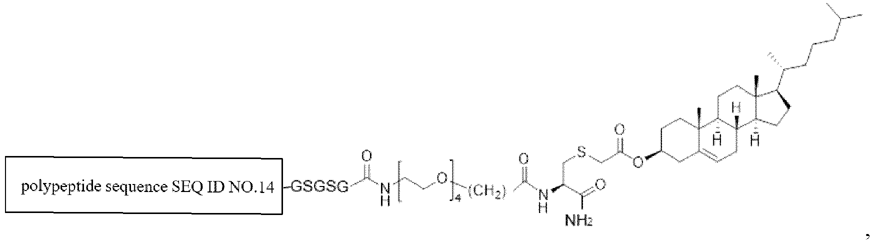

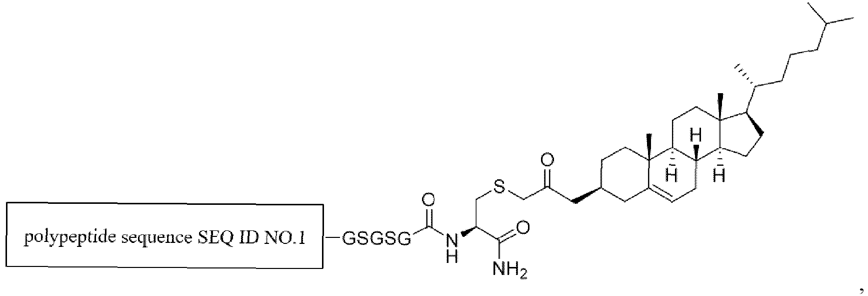

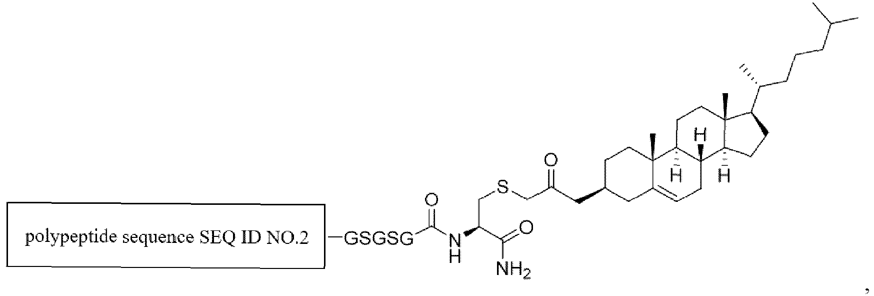

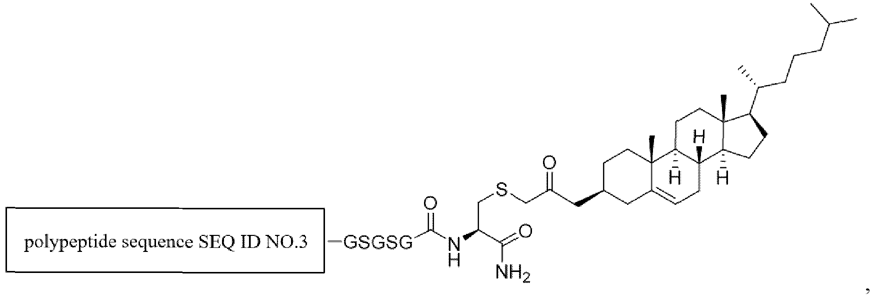

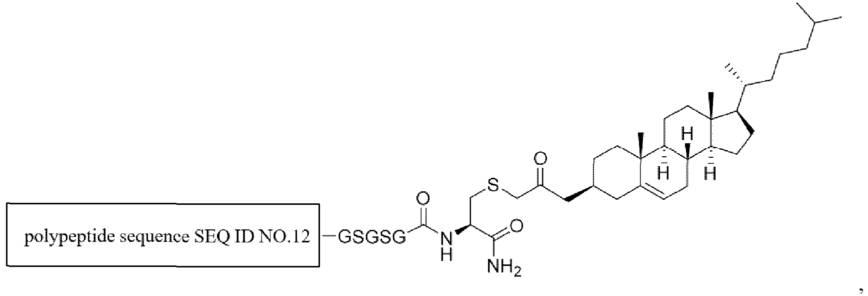

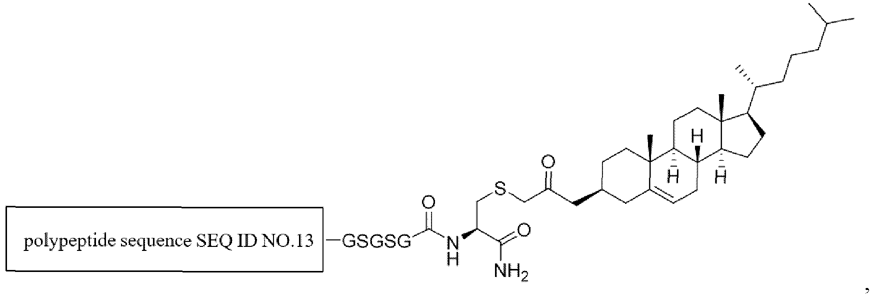

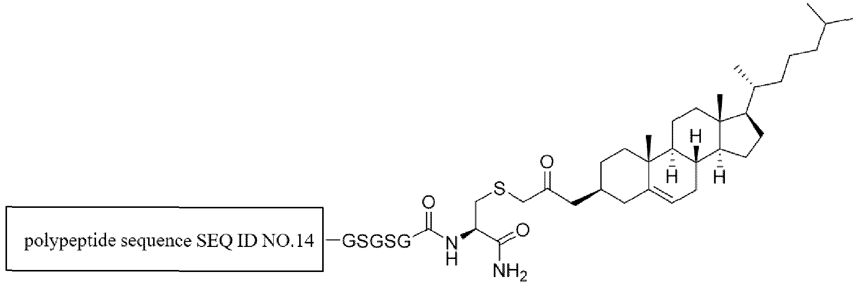

7. A polynucleotide sequence encoding the polypeptide according to claim 1.

8. A vector, comprising the polynucleotide sequence according to claim 7.

9. A cell, comprising the vector according to claim 8.

10. A fusion protein or conjugate, comprising the polypeptide according to claim 1 and/or the polypeptide derivative according to any one of claims 2-5.

11. A pharmaceutical composition, comprising the polypeptide according to claim 1, and/or the polypeptide derivative according to any one of claims 2-5 or a mixture thereof, and/or the fusion protein or conjugate according to claim 10, and a pharmaceutically acceptable carrier and/or excipient, wherein,
preferably, the pharmaceutical composition is in the form of an inhalant, a nasal formulation, an oral formulation, or a parenteral formulation;
preferably, the oral formulation is selected from a tablet, a capsule, a granule, a suspension, a pill, and a solution;
preferably, the parenteral formulation is an injectable or bolus injection formulation; preferably, the pharmaceutical composition is a vaccine composition.

12. Use of the polypeptide according to claim 1, and/or the polypeptide derivative according to any one of claims 2-5 or a mixture thereof, and/or the fusion protein or conjugate according to claim 10 in preparing a medicament for inhibiting a coronavirus or in treating and/or preventing a disease caused by a coronavirus, wherein
preferably, the coronavirus is selected from the SARS-CoV, MERS-CoV, SARS-CoV-2, HCoV-229E, HCoV-OC43, HCoV-NL63, or HCoV-HKU1 prototype or a variant thereof;
preferably, the coronavirus is selected from the SARS-CoV-2 prototype and a SARS-CoV-2 variant;
more preferably, the SARS-CoV-2 variant is selected from Alpha, Beta, Gamma, Delta, Omicron, Kappa, Lambda, and Deltacron;
preferably, the disease caused by a coronavirus is selected from corona virus disease 2019 (COVID-19), severe acute respiratory syndrome, and Middle East respiratory syndrome;
preferably, the dosage form of the medicament is tablet, capsule, dripping pill, aerosol, pill, powder, solution, suspension, emulsion, granule, liposome, transdermal agent, suppository, or lyophilized powder for injection;
preferably, the medicament is preferably administered by: injection, including subcutaneous injection, intravenous injection, intramuscular injection, intraperitoneal injection, intracisternal injection or infusion, etc.; intraluminal administration, e.g., intrarectal, intravaginal and sublingual administration; respiratory administration, e.g., nasal administration; mucosal administration; or topical administration.

13. A method for inhibiting a coronavirus *in vitro,* comprising: administering the polypeptide according to claim 1, and/or the polypeptide derivative according to any one of claims 2-5 or a mixture thereof, and/or the fusion protein or conjugate according to claim 10, and/or the pharmaceutical composition according to claim 11, wherein
preferably, the coronavirus is selected from the SARS-CoV, MERS-CoV, SARS-CoV-2, HCoV-229E, HCoV-OC43, HCoV-NL63, or HCoV-HKU1 prototype or a variant thereof; preferably, the coronavirus is selected from the SARS-CoV-2 prototype and a SARS-CoV-2 variant;
more preferably, the SARS-CoV-2 variant is selected from Alpha, Beta, Gamma, Delta, Omicron, Kappa, Lambda, and Deltacron.

14. A method for preventing or treating a coronavirus infection or a disease caused by a coronavirus in a subject, comprising: administering the polypeptide according to claim 1, and/or the polypeptide derivative according to any one of claims 2-5 or a mixture thereof, and/or the fusion protein or conjugate according to claim 10, and/or the pharmaceutical composition according to claim 11, wherein
preferably, the coronavirus is selected from the SARS-CoV, MERS-CoV, SARS-CoV-2, HCoV-229E, HCoV-OC43, HCoV-NL63, or HCoV-HKU1 prototype or a variant thereof; preferably, the coronavirus is selected from the SARS-CoV-2 prototype and a SARS-CoV-2 variant;
more preferably, the SARS-CoV-2 variant is selected from Alpha, Beta, Gamma, Delta, Omicron, Kappa, Lambda, and Deltacron;
preferably, the disease caused by a coronavirus is selected from corona virus disease 2019 (COVID-19), severe acute respiratory syndrome, and Middle East respiratory syndrome.
